# EUROPEAN PATENT SPECIFICATION

(11) **EP 4 174 070 B1**
(45) Date of publication and mention of the grant of the patent: **29.10.2025**
(21) Application number: 21834246.7
(22) Date of filing: 25.06.2021
(51) Int. Cl.: C07D 487/14, C07D 487/12, A61K 31/522, A61K 31/519, A61P 25/28

(54) **TRICYCLIC PYRIMIDINONE COMPOUND, PREPARATION METHOD THEREFOR, AND COMPOSITION AND USE THEREOF**
TRICYCLISCHE PYRIMIDINONVERBINDUNG, HERSTELLUNGSVERFAHREN DAFÜR SOWIE ZUSAMMENSETZUNG UND VERWENDUNG DAVON
COMPOSÉ DE PYRIMIDINONE TRICYCLIQUE, SON PROCÉDÉ DE PRÉPARATION ET SA COMPOSITION AINSI QUE SON UTILISATION

(30) Priority: 30.06.2020 CN 202010608551; 11.09.2020 CN 202010952714
(43) Date of publication of application: 03.05.2023
(73) Proprietor: Neusco Biotech Limited, Shanghai 201321 (CN)
(72) Inventor: GU, Zhenghua, Shanghai 201321 (CN); WANG, Dongqin, Shanghai 201321 (CN); HU, Youhong, Shanghai 201321 (CN)
(74) Representative: Bandpay & Greuter
(86) International application number: PCT/CN2021/102420
(87) International publication number: WO 2022/001881

(56) References cited:
- WO-A1-2016/011930
- WO-A1-2016/012916
- CN-A- 103 827 116
- CN-A- 105 008 368
- CN-A- 106 536 525

## Description

### Technical field

The invention belongs to the field of medicine, and in particular relates to a tricyclic pyrimidinone compound, its preparation method, its medicinal composition, and its use in medicine.

### BACKGROUND

Lipoprotein-associated phospholipase A2 (Lp-PLA2) is a member of the phospholipase A2 superfamily (Dennis EA, Cao J, Hsu YH, Magrioti V, Kokotos G. Chem Rev. 2011, 111, 6130-6185). It is mainly secreted by monocytes, macrophages, T lymphocytes and chief cells (Stafforini DM, Elstad MR, McIntyre TM, Zimmerman GA, Prescott SM. J Biol Chem. 1990, 265: 9682-9687; Nakajima K, Murakami M, Yanoshita R, Samejima Y, Karasawa K, Setaka M, Nojima S Kudo I. J Biol Chem. 1997, 272, 19708-19713). Phosphatidylcholine sn-2 esters are produced during the oxidation of low-density lipoprotein (LDL). Lp-PLA2 is responsible for the hydrolysis of oxidized phosphatidylcholine sn-2 ester, which then produces oxidized fatty acids and lysophosphatidylcholine (LysoPC) (Caslake MJ, Packard CJ, Suckling KE, Holmes SD, Chamberlain P, Macphee CH. Atherosclerosis. 2000, 150, 413-419; MacPhee CH, Moores KE, Boyd HF, Dhanak D, Ife RJ, Leach CA, Leake DS, Milliner KJ, Patterson RA, Suckling KE, Tew DG, Hickey DM. Biochem J. 1999, 338, 479-487). Both oxidized fatty acids and LysoPC play roles in activating macrophages, increasing oxidative stress, affecting the function of T lymphocytes, and inducing inflammatory responses (Quinn MT, Parthasarathy S, Steinberg D. Proc Natl Acad Sci U S A. 1988, 85, 2805-2809). LysoPCs have been reported to induce the release of multiple cytotoxic inflammatory cytokines (Shi, et al, Atherosclerosis, 2007, 191, 54-62). In addition, LysoPCs have also been involved in the activation of leukocytes, the induction of apoptosis, and the mediation of endothelial dysfunction (Wilensky et al, Current Opinion in Lipidology, 2009, 20, 415-420).

It has been reported that plasma level of Lp-PLA2 is associated with cardiovascular diseases (Fitzpatrick AL, Irizarry MC, Cushman M, Jenny NS, Chi GC, Koro C. Atherosclerosis. 2014, 235, 384-391), diabetic macular edema (DME) (Staurenghi G, Ye L, Magee MH, Danis RP, Wurzelmann J, Adamson P, McLaughlin MM, Darapladib DMES G. Ophthalmology. 2015, 122, 990-996), and prostate cancer (Bertilsson H, Tessem MB, Flatberg A, Viset T, Gribbestad I, Angelsen A, Halgunset J. Clin Cancer Res. 2012, 18, 3261-3269).

Alzheimer's disease (AD) is a chronic neurodegenerative disease that results in decreasd cognitive abilities, mood swings, irreversible memory loss, disorientation, speech impairment, and loss of self-protection (Hardy J, et al. Science 2002, 297, 353-356). Alzheimer's disease usually starts slowly and gets worse over time, which is the cause of 60 % to 70 % of dementia cases and affects about 6 % of the population over 65 years old. AD patients will gradually withdraw from family and society, rely more and more on help, and eventually progress to death. AD is one of the most costly diseases in developed countries and also with high costs in other countries. These costs will increase dramatically, especially as aging becomes a major societal issue. Needless to say, AD is a complex disease involving multiple factors. Although the etiology of AD has not been fully elucidated, it is clear that several factors are involved in the initiation and progression of the disease, including aggregated tau protein and Aβ peptide, oxidative stress, and neuroinflammation (Echeverria V, Yarkov A, Aliev G. Prog Neurobiol. 2016, 144, 142-157). The current research and development of AD drug is maily focused on targets of Aβ amyloidosis and tau (Chiang K, Koo EH. Annu Rev Pharmacol Toxicol. 2014, 54, 381-405; Awasthi M, Singh S, Pandey VP, Dwivedi UN. J Neurol Sci. 2016, 361, 256-271). However, despite strong preclinical data, results from late-stage clinical trials have so far failed to demonstrate clinical efficacy. These disappointing results suggest that other mechanisms of neuropathology, such as oxidative stress and neuroinflammation, may have to be explored for AD therapy.

Elevated levels of Lp-PLA2 in plasma increase the risk of dementia, including AD (Van Oijen, et al. Annals of Neurology, 2006, 59,139). In addition to vascular dementia and mixed dementia, high oxidized LDL levels have been found in AD patients (Maher-Edwards G, De'Ath J, Barnett C, Lavrov A, Lockhart A, Alzheimer's & Dementia: Translational Research & Clinical Interventions. 2015, 1, 131-140; Kassner et al. Current Alzheimer Research, 2008, 5, 358-366; Dildar, et al., Alzheimer Dis Assoc Disord, 24, April-June (2010); Sinem, et al. Current Alzheimer Research, 2010, 7, 463-469). Neuroinflammation and upregulation of multiple inflammatory cytokines were also found in AD patients (Colangelo, et al., Journal of Neuroscience Research, 2002, 70, 462-473; Wyss-Coray, Nature Medicine, 2006, 12, Sept.).

Based on all these findings, Lp-PLA2 is a potential target for the treatment of AD, and this is further confirmed by the clinical results of the Lp-PLA2 inhibitor Rilapladib for AD patients (Maher-Edwards G, De'Ath J, Barnett C, Lavrov A, Lockhart A, Alzheimer's & Dementia: Translational Research & Clinical Interventions. 2015, 1, 131-140).

Glaucoma and age-related macular degeneration (AMD) are retinal neurodegenerative diseases. Buschini et al reported that inflammation, including TNF-α signaling, may play an important role in the pathogenesis of glaucoma and AMD (Buschini et al, Progress in Neurobiology, 2011, 95, 14-25; Tezel, Progress in Brain Research, vol. 173, ISSN0079-6123, Chapter 28). Additionally, Shi et al demonstrated that Lp-PLA2 inhibitors can block the release of inflammatory cytokines (Shi, et al, Atherosclerosis, 2007, 191, 54-62). Inhibition of Lp-PLA2 is potential treatment for glaucoma and AMD.

A number of Lp-PLA2 inhibitors have been reported, including β-lactams (Tew DG, Boyd HF, Ashman S, Theobald C, Leach CA. Biochemistry. 1998, 37, 10087-10093), oximes (Jeong TS, Kim MJ, Yu H, Kim HS, Choi JK, Kim SS, Lee WS. Bioorg Med Chem Lett. 2005, 15, 1525-1527; Jeong HJ, Park YD, Park HY, Jeong IY, Jeong TS, Lee WS. Bioorg Med Chem Lett. 2006, 16, 5576-5579), amides of xanthuric acid (Lin EC, Hu Y, Amantea CM, Pham LM, Cajica J, Okerberg E, Brown HE, Fraser A, Du L, Kohno Y, Ishiyama J, Kozarich JW, Shreder KR. Bioorg Med Chem Lett. 2012, 22, 868-871; Hu Y, Lin EC, Pham LM, Cajica J, Amantea CM, Okerberg E, Brown HE, Fraser A, Du L, Kohno Y, Ishiyama J, Kozarich JW, Shreder KR. Bioorg Med Chem Lett. 2013, 23, 1553-1556), and carbamates (Nagano JM, Hsu KL, Whitby LR, Niphakis MJ, Speers AE, Brown SJ, Spicer T, Fernandez-Vega V, Ferguson J, Hodder P, Srinivasan P, Gonzalez TD, Rosen H, Bahnson BJ, Cravatt BF. Bioorg Med Chem Lett. 2013, 23, 839-843).

The Lp-PLA2 inhibitor Darapladib has been reported as a potential therapy against atherosclerosis and DME (Magrioti V, Kokotos G. Expert Opin Ther Pat. 2013; 23: 333-344).

### SUMMARY

The present inventors have found that Lp-PLA2 inhibitors play an important role in the treatment of neurodegenerative related diseases, such as Alzheimer's disease (AD), glaucoma and age-related macular degeneration (AMD), or cardiovascular diseases including atherosclerosis. For this reason, the present inventors have developed a novel type of Lp-PLA2 inhibitor, tricyclic pyrimidinone compound

The tricyclic pyrimidinone compound is a compound having a structure represented by Formula (I) or a pharmaceutically acceptable salt thereof, 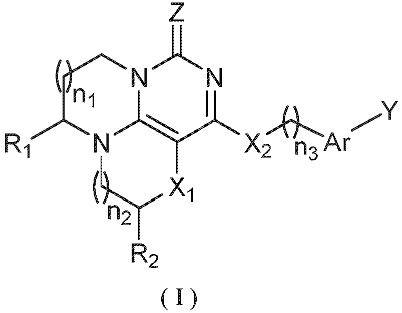 wherein
n₁, n₂, and n₃ are each independently 0, 1, or 2;
R₁ and R₂ are each independently selected from -H, hydroxyl, cyano, halogen, alkyl, deuterated alkyl, hydroxyalkyl, haloalkyl, cycloalkyl, alkoxy, haloalkoxy, deuterated alkoxy;
X₁ and X₂ are each independently selected from alkylene, -O-, -S-, or -NR'-,
R' is selected from -H, alkyl, deuterated alkyl, or cycloalkyl;
Ar is an arylene group or a heteroarylene group, wherein hydrogen atoms in the arylene or heteroarylene are optionally substituted by 0, 1 or more substituents, and the substituents are each independently selected from halogen, alkyl, deuteroalkyl, haloalkyl, alkoxy, deuteroalkoxy, haloalkoxy, hydroxy, hydroxyalkyl, cyano, amino, monoalkyl- or dialkyl-substituted amino, nitro, carboxyl, aldehyde, cycloalkyl, heterocyclyl, aryl, or heteroaryl;
Y is -H, halogen, alkyl, haloalkyl, haloalkoxy, cycloalkyl, alkoxy, deuterated alkyl, deuterated alkoxy, -OAr', -SAr', -NH-Ar', -NMe-Ar', -NR", or -R"'-Ar';
Ar' is selected from aryl or heteroaryl, wherein hydrogen atoms in the aryl or heteroaryl are optionally substituted with one or more substituents, the substituents are each independently selected from halogen, alkyl, deuterated alkyl, haloalkyl, alkoxy, deuterated alkoxy, hydroxy, hydroxyalkyl, haloalkoxy, cyano, amino, nitro, carboxyl, aldehyde, cycloalkyl, heterocyclyl, aryl, or heteroaryl;
R" is alkyl;
R'" is alkylene;
Z is O or S.

Optionally, halogens in the "halogen" "haloalkyl" and "haloalkoxy" are each independently selected from F, Cl, Br, or I;
optionally, alkyls in the "alkyl" "deuterated alkyl" "hydroxyalkyl" "haloalkyl" "haloalkoxy", "alkoxy" and "mono- or di-alkyl substituted amino" are each independently C₁-C₁₀ linear or branched alkyl; optionally each independently C₁-C₇ linear or branched alkyl; optionally each independently C₁-C₄ linear or branched alkyl; and optionally selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl;
optionally, "alkylenes" are each independently C₁-C₁₀ linear or branched alkylene; optionally each C₁-C₇ linear or branched alkylene; optionally each C₁-C₅ linear or branched alkylene; and optionally each selected from methylene, ethylene, n-propylene, iso-propylene, n-butylene, iso-butylene, tert-butylene, sec-butylene, n-pentylene, 1-methylbutylene, 2-methylbutylene, 3-methylbutylene, isopentylene, 1-ethylpropylene, neopentylene, n-hexylene, 1-methylpentylene, 2-methylpentylene, 3-methylpentylene, isohexylene, 1,1-dimethylbutylene, 2,2-dimethylbutylene, 3,3-dimethylbutylene, 1,2-dimethylbutylene, 1,3-dimethylbutylene, 2,3-dimethylbutylene, 2-ethylbutylene, n-heptylene, 2-methylhexylene, 3-methylhexylene, 2,2-dimethylpentylene, 3,3-dimethylpentylene, 2,3-dimethylpentylene, 2,4-dimethylpentylene, 3-ethylpentylene, or 2,2,3-trimethylbutylene;
optionally, "cycloalkyl" is C₃-C₁₀ monocyclic or bicyclic cycloalkyl, optionally C₃-C₇ monocyclic cycloalkyl, and optionally cyclopropyl, cyclobutyl, cyclopentyl, cyclohexyl, or cycloheptyl;
optionally, "heterocyclyl" is 3- to 10-membered non-aromatic heterocycle ring containing 1, 2, or 3 heteroatoms selected from N, O, and S; optionally 3- to 10-membered non-aromatic ring containing 1 or 2 heteroatoms selected from N and O; optionally 3- to 6-membered non-aromatic ring containing 1 or 2 heteroatoms selected from N and O; optionally 3- to 10-membered non-aromatic ring containing 1 or 2 heteroatoms selected from N and S; and optionally 3- to 6-membered non-aromatic ring containing 1 or 2 heteroatoms selected from N and S;
optionally, "aryl" is 6- to 10-membered aryl; optionally phenyl or naphthyl, and optionally phenyl, 1-naphthyl, or 2-naphthyl;
optionally, "arylene" is 6- to 10-membered arylene; and optionally phenylene or naphthylene;
optionally, "heteroaryl" is 5- to 10-membered heteroaryl ring containing 1-3 heteroatoms selected from N, O, and S; optionally 5- to 10-membered heteroaryl ring containing 1-2 heteroatoms selected from N, O, and S; optionally the heteroaryl ring is selected from pyridine ring, pyrrole ring, pyrazole ring, pyrimidine ring, pyrazine ring, pyridazine ring, thiophene ring, and furan ring; optionally selected from pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, pyrazin-3-yl, indolyl, isoindolyl, indazolyl, indolizinyl, purinyl, quinolizinyl, quinolinyl, isoquinolinyl, cinnolinyl, phthalazinyl, naphthyridinyl, quinazolinyl, quinoxalinyl, thieno[2,3-b] furanyl, furo[3,2-b]-pyranyl, pyrido[2,3-d]oxazinyl, pyrazolo[4,3-d]oxazolyl, imidazo[4,5-d]thiazolyl, pyrazino[2,3-d]pyridazinyl, imidazo[2,1-b]thiazolyl, imidazo[1,2-b][1,2,4]triazinyl, benzothienyl, benzoxazolyl, benzimidazolyl, benzothiazolyl, benzoxepinyl, benzoxazinyl, benzofuranyl, benzotriazolyl, pyrrolo[2,3-b]pyridyl, pyrrolo[3,2-c]pyridinyl, pyrrolo[3,2-b]pyridyl, imidazo[4,5-b]pyridyl, imidazo[4,5-c]pyridyl, pyrazolo[4,3-d]pyridyl, pyrazolo[4,3-c]pyridyl, pyrazolo[3,4-c]pyridinyl, pyrazolo[3,4-d]pyridyl, pyrazolo[3,4-b] pyridinyl, imidazo[1,2-a]pyridinyl, pyrazolo[1,5-a]pyridinyl, pyrrolo[1,2-b]pyridazinyl, imidazo[1,2-c]pyrimidinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazolo[2,3-b]pyrazinyl, or pyrimido[4,5-d]pyrimidinyl; and is optionally selected from pyridin-2-yl, pyridin-3-yl, pyridin-4-yl, pyrimidin-2-yl, pyrimidin-4-yl, or pyrimidin-5-yl;
optionally, "heteroarylene" is 5- to 10-membered heteroarylene ring containing 1-3 heteroatoms selected from N, O, and S; optionally 5- to 10-membered heteroaromatic ring containing 1-2 heteroatoms selected from N, O, and S; and optionally the heteroarylene ring is selected from pyridine ring, pyrrole ring, pyrazole ring, pyrimidine ring, pyrazine ring, pyridazine ring, thiophene ring, furan ring

Optionally, n₁, n₂, and n₃ are each independently 0, 1, or 2.

Optionally, n₁ is 0 or 1.

Optionally, n₁ is 0.

Optionally, n₂ is 0 or 1.

Optionally, n₂ is 1.

Optionally, n₃ is 1.

Optionally, R₁ and R₂ are each independently selected from -H, fluorine, chlorine, bromine, hydroxyl, cyano, C₁-C₇ alkyl (such as methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), C₁-C₃ deuteroalkyl (such as -CD₃, -C₂D₅, or -C₃D₇), C₁-C₃ deuteroalkoxyl (such as -OCD₃, - OC₂D₅, or -OC₃D₇), haloalkyl, haloalkoxyl, cyclopropanyl, cyclobutanyl, cyclopentanyl; optionally, R₁ is -H or -CH₃; and optionally, R₁ is-H, R₂ is-H;
optionally, X₁ and X₂ are each independently selected from C₁-C₇ alkylene (optionally, - CH₂-, ethylene, n-propylene, isopropylene, n-butylene, or isobutylene), -O-, -S-, or -NR'-; optionally, X₁ is -CH₂-, or -O-; optionally, X₁ is -O-; optionally, X₂ is -O-;
optionally, R' is selected from -H, C₁-C₇ alkyl (optionally, methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), deuterated alkyl (optionally, -CD₃, -C₂D₅, or -C₃D₇), or C₃-C₆ cycloalkyl (optionally, cyclopropanyl, cyclobutanyl, cyclopentanyl, or cyclohexanyl);
optionally, Ar is phenylene or pyridyl, wherein hydrogen atoms in the phenylene or pyridyl are optionally substituted with 0, 1, 2, or 3 substituents, the substituents are each independently selected from F, Cl, Br, I, -CN, -Me, -C₂H₅, cyclopropyl, -CD₃, -OMe, -OCD₃, -CF₃, or -OCF₃; optionally, Ar is phenylene, wherein the hydrogen atom in the phenylene is optionally substituted by 2 substituents, and the substituent is F;
optionally, Y is -H, -F, -Cl, -Br, methyl, ethyl, n-propyl, isopropyl, -CD₃, -CF₃, -CH₂CF₃, - OCF₃, -OCHF₂, -OCH₂F, cyclopropyl, cyclobutyl, cyclopentyl, -OCH₃, -OCD₃, -OC₂H₅, -OC₃H₇, or -OAr';
optionally, Y is H, halogen, or -OAr'; and optionally, Y is H, -F, or -OAr';
Ar' is selected from phenyl, pyridyl, pyrimidyl, pyrrolyl, pyrazolyl, thienyl or quinolinyl, and the phenyl, pyridyl, pyrimidyl, pyrrolyl, pyrazolyl, thienyl or quinoline
optionally, Ar' is selected from phenyl, pyridyl, pyrimidyl, pyrrolyl, pyrazolyl, thienyl or quinolinyl, wherein hydrogen atoms in the phenyl, pyridyl, pyrimidyl, pyrrolyl, pyrazolyl, thienyl or quinolinyl ring are each independently optionally substituted with 1, 2, or 3 substituents, the substituents are each independently selected from F, Cl, Br, -CN, C₁-C₇ alkyl (optionally, methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), -CD₃, C₁-C₆ haloalkyl, -OCH₃, -OCD₃, -OC₂H₇, -OC₃H₇, C₁-C₆ haloalkoxyl, or C₃-C₆ cycloalkyl (optionally, cyclopropanyl, cyclobutanyl, cyclopentanyl, or cyclohexanyl);
optionally, Ar' is selected from phenyl, pyridin-3-yl, pyridin-4-yl, or pyrimidin-5-yl, and is optionally substituted with 1 or 2 substituents, the substituents are selected from F, Cl, -CH₃, - CF₃, or-OCF₃;
optionally, Z is O.

Optionally, of a compound of Formula (I) or a pharmaceutically acceptable salt thereof, the compound of Formula (I) is selected from the following compounds:

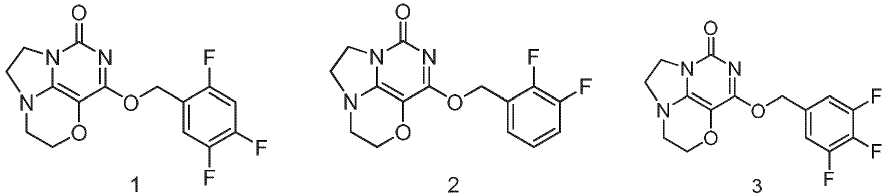

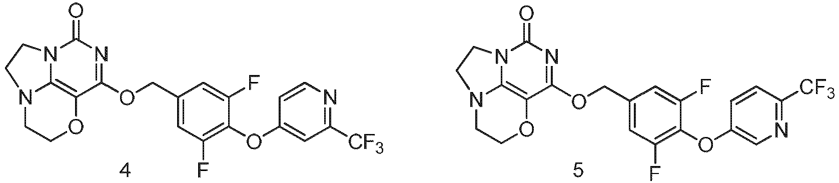

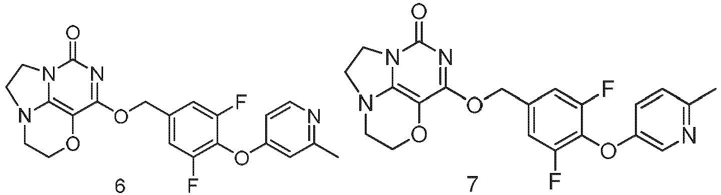

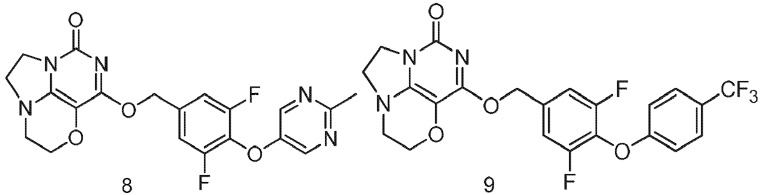

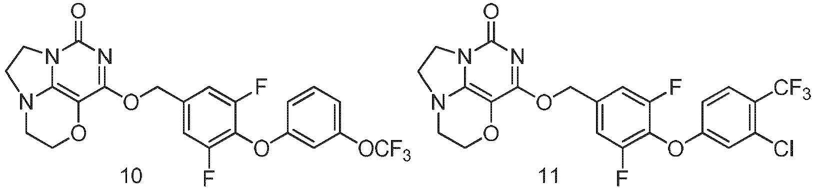

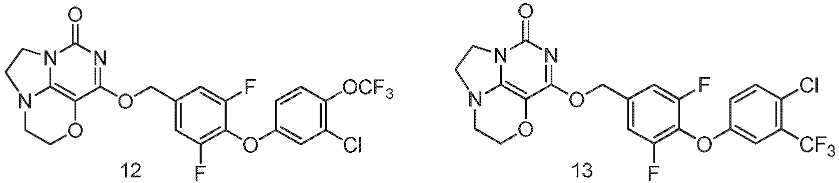

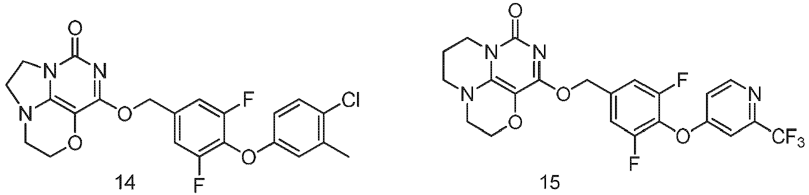

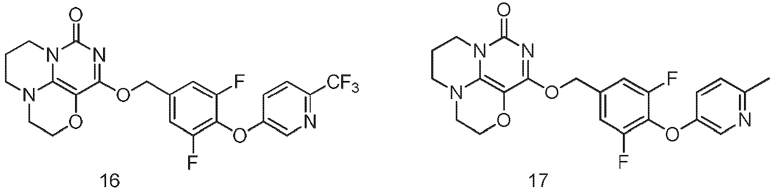

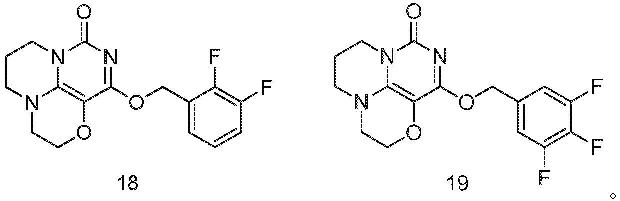

Optionally, the compound of formula (I) or its tautomers, mesoforms, racemates, enantiomers, diastereoisomers, or mixtures thereof, or pharmaceutically acceptable salts, include anionic salts and cationic salts of compounds of formula (I);
optionally, the pharmaceutically acceptable salt includes alkali metal salt, alkaline earth metal salt, or ammonium salt of the compound of Formula (I); optionally, the alkali metal includes sodium, potassium, lithium, or cesium, and the alkaline earth metal includes magnesium, calcium, or strontium;
optionally, the pharmaceutically acceptable salt comprises a compound of formula I or its tautomer, mesoform, racemate, enantiomer, diastereoisomer, or a mixture with an organic base;
optionally, the organic base includes trialkylamine, pyridine, quinoline, piperidine, imidazole, picoline, dimethylaminopyridine, dimethylaniline, N-alkylmorpholine, 1,5-diazabicyclo[ 4.3.0] nonene-5, 1,8-diazabicyclo[5.4.0]undecene-7, 1,4-diazabicyclo[2.2.2]octane; alternatively, all Described trialkylamine comprises trimethylamine, triethylamine, N-ethyldiisopropylamine; Optionally, described N-alkylmorpholine comprises N-methylmorpholine;
optionally, the pharmaceutically acceptable salt comprises a compound of formula I or its tautomer, mesoform, racemate, enantiomer, diastereoisomer, or a mixture with an an acid;
optionally, the acid includes inorganic acid, organic acid; optionally, the inorganic acid includes hydrochloric acid, hydrobromic acid, hydroiodic acid, sulfuric acid, nitric acid, phosphoric acid, carbonic acid; optionally, the organic acid includes formic acid, acetic acid, Propionic acid, oxalic acid, malonic acid, succinic acid, fumaric acid, maleic acid, lactic acid, malic acid, citric acid, citric acid, tartaric acid, carbonic acid, picric acid, methanesulfonic acid, ethanesulfonic acid, p-toluenesulfonic acid acid, glutamic acid, pamoic acid

In another aspect, there is provided a preparation method of a compound of Formula (I) or a pharmaceutically acceptable salt thereof, comprising the step of reacting a compound of Formula (II) with a compound of Formula (III) to produce the compound of Formula (I):

In another aspect, there is provided a preparation method of compound of formula (I) or its tautomer, mesomer, racemate, enantiomer, diastereoisomer, or a mixture thereof, or the pharmaceutically acceptable salt. The method is shown as the below reaction scheme:

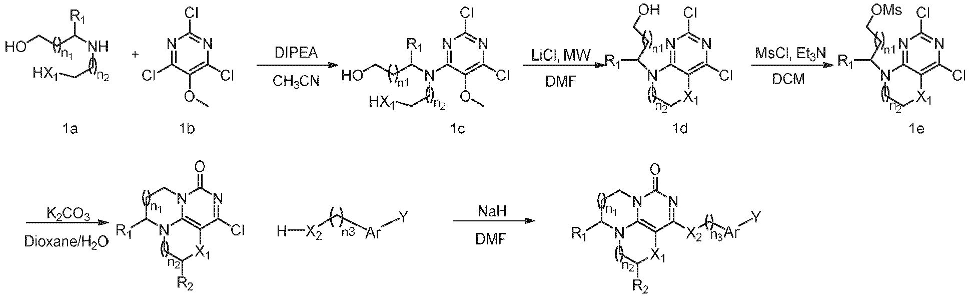

In each of the formulas in the preparation method described above, n₁, n₂, n₃, R₁, R₂, X₁, X₂, Ar, and Y are defined as above.

There are no specific limitations for the above reaction conditions. All the reactions can be carried out under conventional conditions.

In another aspect, there is provided a pharmaceutical composition, comprising a therapeutically effective amount of one or more of the compound of Formula (I) or a pharmaceutically acceptable salt thereof, and optionally, a pharmaceutically acceptable excipient(s).

In another aspect, a pharmaceutical composition is provided, which comprises a therapeutically effective amount of the above-mentioned compound of formula (I) or its tautomer, mesoform, racemate, enantiomer, diastereomer One or more of isomers, or a mixture thereof, or a pharmaceutically acceptable salt thereof, and optionally a pharmaceutically acceptable carrier.

Optionally, the dosage form of the pharmaceutical composition includes oral, rectal, or parenteral formulation;
optionally, the oral formulation includes solid or liquid formulation;
optionally, the solid formulation includes tablet, powder, granule, or capsule;
optionally, the liquid formulation includes aqueous or oily suspension, or syrup;
optionally, the parenteral formulation includes solution for injection, or aqueous or oily suspension.

In another aspect, there is provided the above-mentioned compound of formula (I) or its tautomer, mesomer, racemate, enantiomer, diastereoisomer, or mixture thereof, or its pharmaceutically acceptable Salt, or the above-mentioned pharmaceutical composition in the preparation of Lp-PLA2 inhibitor.

In another aspect, there is provided the above-mentioned compound of formula (I) or its tautomer, mesomer, racemate, enantiomer, diastereoisomer, or mixture thereof, or its pharmaceutically acceptable Salt, or the above-mentioned pharmaceutical composition, in the preparation of a medicament for treatment of neurodegeneration-related diseases;
optionally, the neurodegeneration-related diseases include Alzheimer's disease (AD), glaucoma, and age-related macular degeneration (AMD).

In another aspect, there is provided the above-mentioned compound of formula (I) or its tautomer, mesomer, racemate, enantiomer, diastereoisomer, or mixture thereof, or its pharmaceutically acceptable Salt, or the above-mentioned pharmaceutical composition, in the preparation of a medicament for the treatment of cardiovascular diseases, diabetic macular edema (DME), or prostate diseases
optionally, the cardiovascular diseases include atherosclerosis.

Beneficial effects of the present disclosure are as follows:
The compound of Formula (I) is an tricyclic pyrimidinone compound as an novel Lp-PLA2 inhibitor. It can be used to treat neurodegenerative related diseases such as Alzheimer's disease (AD), glaucoma and age-related macular degeneration (AMD), or cardiovascular diseases including atherosclerosis.

### DETAILED DESCRIPTION

The present invention is further illustrated by the following examples. It should be understood that the embodiments herein are only used to illustrate the present invention, and do not limit the scope of the present invention in any way.

The starting materials of the present invention can be synthesized by a method known in the art, or be purchased from ABCR GmbH & Co. KG, Acros Organics, Aldrich Chemical Company, Accela ChemBio Inc., and Darry Chemicals, among other companies.

Unless otherwise specified, the solution in the examples refers to an aqueous solution.

Unless otherwise specified, the temperature in the examples at which the reaction is carried out is room temperature, e.g., 20 °C to 30 °C.

### Example 1 Preparation of Compound 1

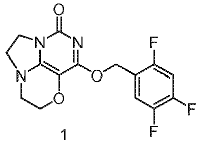

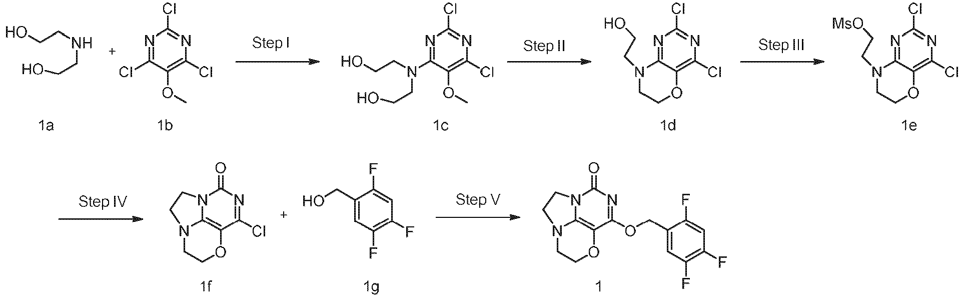

### Step I: Preparation of compound 1c

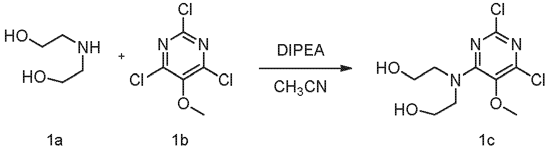

At room temperature, 2,4,6-trichloro-5-methoxypyrimidine **1b** (1.5 g, 7.03 mmol), diethanolamine **1a** (1.1 g, 10.5 mmol) and diisopropylethylamine (1.36 g, 10.54 mmol ) were dissolved in acetonitrile (50 mL), stirred and reacted for 3 h, concentrated under reduced pressure. Purification via silica gel column chromatography with an eluent system (dichloromethane/methanol=20/1) afforded the title compound **1c** (1.6 g, Yield: 81.0%) as a white solid.
¹H NMR (400 MHz, CDCl₃) δ 3.94 - 3.89 (m, 4H), 3.88 - 3.83 (m, 4H), 3.77 (s, 3H), 3.17 (s, 2H).

### Step II: Preparation of compound 1d

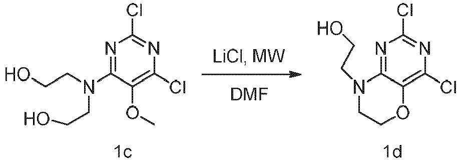

At room temperature, 2,2'-((2,6-dichloro-5-methoxypyrimidine-4-yl)azanediyl)diethanol 1c (1.0 g, 3.54 mmol), and lithium chloride (0.75 g, 17.73 mmol) were dissolved in N,N-dimethylformamide (5 mL), heated in microwave at 130°C for 1 h, concentrated under reduced pressure, extracted with ethyl acetate (60 mL×3). The combined organic phases were washed with a saturated aqueous solution of sodium chloride, dried over anhydrous sodium sulfate, filtered to remove desiccant, concentrated under reduced pressure. Purification via silica gel column chromatography with an eluent system (dichloromethane/methanol=20/1) afforded the title compound **1d** (0.39 g, Yield: 44.0%) as a white solid.

¹H NMR (400 MHz, CDCl₃) δ 4.32 - 4.27 (m, 2H), 3.92 (m, 2H), 3.83 - 3.76 (m, 2H), 3.76 - 3.69 (m, 2H).

### Steps III and IV: Preparation of compound 1f

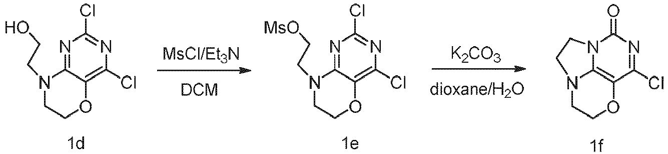

At room temperature, 2-(2,4-dichloro-6hydro-pyrimido[5,4-b][1,4]oxazin-8(7H)-yl)ethanol **1d** (0.77 g, 3.1 mmol) and Triethylamine (0.94 g, 9.3 mmol) were dissolved in dichloromethane (30 mL), then methanesulfonyl chloride (0.39 g, 3.4 mmol) was added dropwise at 0°C. The reaction was stirred and reacted at 0°C for 1 h, concentrated and proceeded directly to the next reaction. The crude product was dissolved in a mixed solvent of 1/1 dioxane/water (60 mL), followed by addition of potassium carbonate (1.3 g, 9.3 mmol) at room temperature. The reaction mixture was stirred at 90°C overnight, concentrated under reduced pressure. Purification via silica gel column chromatography with an eluent system (dichloromethane/methanol=10/1) afforded the title compound **1f** (0.38 g, Yield: 57.4%) as a white solid.

¹H NMR (400 MHz, MeOD) δ 4.32 - 4.25 (m, 2H), 4.25 - 4.17 (m, 2H), 3.99 (m, 2H), 3.60 - 3.52 (m, 2H).

### Step V: Preparation of compound 1

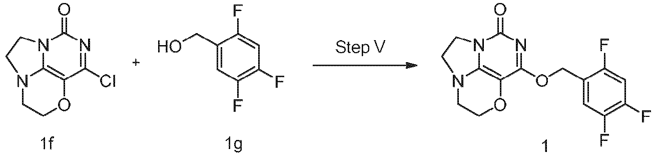

To a solution of (2,4,5-trifluorophenoxy)methanol **1g** (39 mg, 0.24 mmol) in dry *N,N-*dimethylformamide (5 mL) was added sodium hydride (60 % in mineral oil, 16 mg, 0.4 mmol) at 0 °C, and stirred at room temperature for 5 min. Then compound **1f** (43 mg, 0.20 mmol) was added, and stirred for 1 h, quenched with a small amount of water. Purification via silica gel column chromatography with an eluent system (dichloromethane/methanol=10/1) afforded the title compound 1 (13.6 mg, 20 %) as a white solid.

¹H NMR (400 MHz, CDCl₃) δ 7.30 (m, 1H), 6.96 (m, 1H), 5.43 (s, 2H), 4.32 - 4.22 (m, 2H), 4.20 (m, 2H), 3.78 (m, 2H), 3.48 - 3.39 (m, 2H). MS (ESI): m/z 340.1 [M+H]⁺_{∘}

### Example 2 Preparation of Compound 2

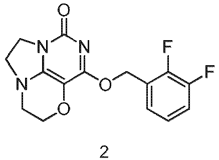

To a solution of (2,3-Difluorophenyl)methanol (35mg , 0.24 mmol) in dry *N,N-*dimethylformamide (5 mL) was added sodium hydride (60 % in mineral oil, 16 mg, 0.4 mmol) at 0 °C, and stirred at room temperature for 5 min. Then compound **1f** (43 mg, 0.20 mmol) was added, and stirred for 1 h, quenched with a small amount of water. Purification via silica gel column chromatography with an eluent system (dichloromethane/methanol=10/1) afforded the title compound 2 (15 mg, 23.3 %) as a white solid.

¹H NMR (400 MHz, CDCl₃) δ 7.45 (m, 1H), 6.96 - 6.82 (m, 2H), 5.43 (s, 2H), 4.33 - 4.22 (m, 2H), 4.19 (m, 2H), 3.77 (m, 2H), 3.46 - 3.39 (m, 2H). MS (ESI): m/z 322.1 [M+H]⁺_{∘}

### Example 3 Preparation of Compound 3

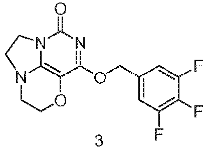

To a solution of (3,4,5-Trifluorophenyl)methanol (39mg, 0.24 mmol) in dry *N,N-*dimethylformamide (5 mL) was added sodium hydride (60 % in mineral oil, 16 mg, 0.4 mmol) at 0 °C, and stirred at room temperature for 5 min. Then compound **1f** (43 mg, 0.20 mmol) was added, and stirred for 1 h, quenched with a small amount of water. Purification via silica gel column chromatography with an eluent system (dichloromethane/methanol=10/1) afforded the title compound 3 (11.5 mg, 17 %) as a white solid.

¹H NMR (400 MHz, CDCl₃) δ 7.31 (m, 2H), 5.42 (s, 2H), 4.31 - 4.22 (m, 2H), 4.20 (m, 2H), 3.78 (m, 2H), 3.46 - 3.38 (m, 2H). MS (ESI): m/z 340.1 [M+H]⁺_{∘}

### Example 4 Preparation of Compound 4

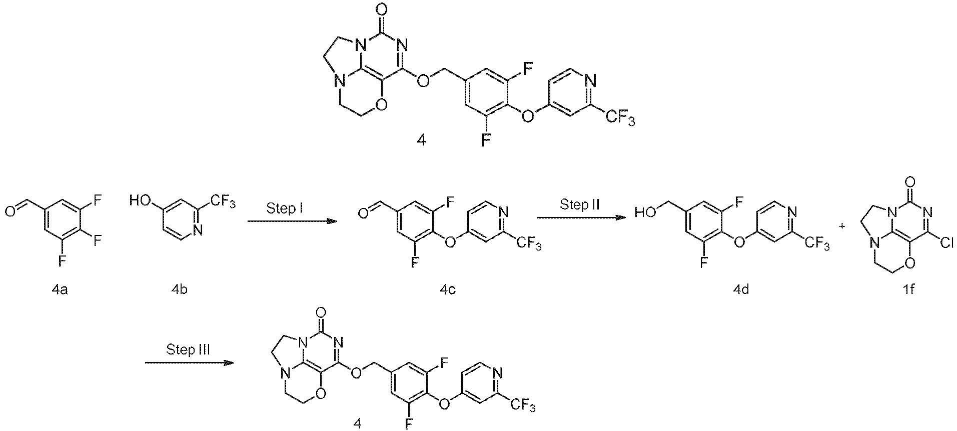

### Step I: Preparation of compound 4c

2-(Trifluoromethyl)pyridin-4-ol 4b (0.85 g, 5.2 mmol), 3,4,5-trifluorobenzaldehyde 4a (1 g, 6.2 mmol) and potassium carbonate (0.93 g, 6.76 mmol) were dissolved in N,N-dimethylformamide (DMF) (30 mL) at room temperature. The reaction mixture was stirred at 90°C for 1 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=5/1) afforded the title compound 4c (1.47 g, yield: 93.2%) as a yellow solid.

¹H NMR (400 MHz, CDCl₃) δ 9.97 (s, 1H), 8.65 (m, 1H), 7.63 (m, 2H), 7.27 (m, 1H), 7.01 (m, 1H)_{∘}

### Step II: Preparation of compound 4d

At room temperature, 3,5-difluoro-4-((2-(trifluoromethyl)pyridin-4-yl)oxy)benzaldehyde 4c (1.47 g, 4.85 mmol) was dissolved in ethanol (50 mL), then NaBH₄ (184 mg, 4.84 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous Sodium sulfate, filtered to remove desiccant. The filtrate was concentrated under reduced pressure. Purification via silica gel column chromatography with an eluent system (petroleum ether/ethyl acetate=2/1) afforded the title compound 4d (1.04 g, yield: 70.3%) as a white solid.

¹H NMR (400 MHz, CDCl₃) δ 8.59 (m, 1H), 7.24 (m, 1H), 7.11 (m, 2H), 6.99 (m, 1H), 4.75 (m, 2H), 2.19 (m, 1H)_{∘}

### Step III: Preparation of compound 4

To a solution of (3,5-Difluoro-4-((2-(trifluoromethyl)pyridin-4-yl)oxy)phenyl)methanol 4d (68mg, 0.22 mmol) in dry N,N-dimethylformamide (5 mL) was added sodium hydride (60 % in mineral oil, 16 mg, 0.4 mmol) at 0 °C, and stirred at room temperature for 5 min. Then compound **1f** (43 mg, 0.20 mmol) was added, and stirred for 1 h, quenched with a small amount of water. Purification via silica gel column chromatography with an eluent system (dichloromethane/methanol=10/1) afforded the title compound 4 (31 mg, 32.1 %) as a white solid.

¹H NMR (400 MHz, CDCl₃) δ 8.58 (m, 1H), 7.24 (m, 1H), 7.16 (m, 2H), 6.96 (m, 1H), 5.45 (s, 2H), 4.32 - 4.23 (m, 2H), 4.19 (m, 2H), 3.78 (m, 2H), 3.46 - 3.37 (m, 2H). MS (ESI): m/z 482.9 [M+H]⁺_{∘}

### Example 5 Preparation of Compound 5

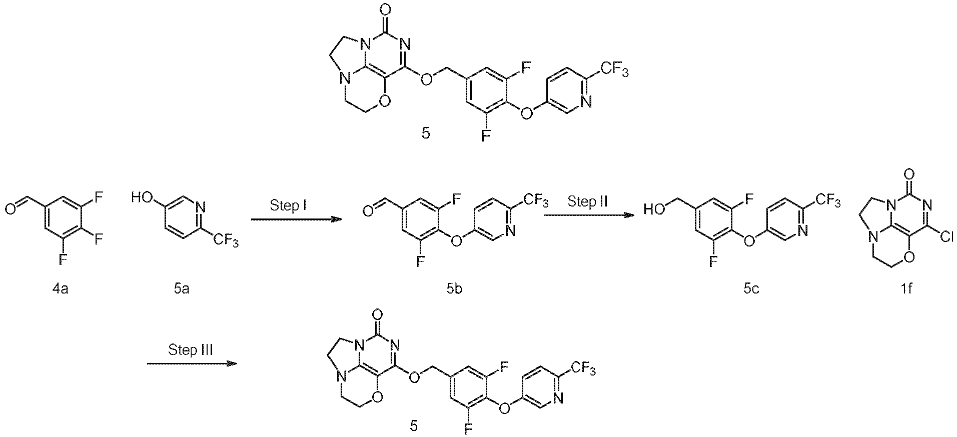

### Step I: Preparation of compound 5b

6-(trifluoromethyl)pyridin-3-ol **5a** (0.85 g, 5.2 mmol), 3,4,5-trifluorobenzaldehyde **4a** (1 g, 6.2 mmol) and potassium carbonate (0.93 g, 6.76 mmol) were dissolved in N,N-dimethylformamide (DMF) (30 mL) at room temperature. The reaction mixture was stirred at 90°C for 1 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=5/1) afforded the title compound **5b** (1.34 g, yield: 85.0%) as a yellow solid.

### Step II: Preparation of compound 5c

At room temperature, 3,5-Difluoro-4-((6-(trifluoromethyl)pyridin-3-yl)oxy)benzaldehyde **5b** (1.34 g, 4.4 mmol) was dissolved in ethanol (50 mL), then NaBH₄ (167 mg, 4.4 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=2/1) afforded the title compound **5c** (0.77 g, yield: 57.3%) as a colorless oil.

¹H NMR (400 MHz, CDCl₃) δ 8.46 (m, 1H), 7.63 (m, *m* 1H), 7.30 (m, 1H), 7.09 (m, 2H), 4.73 (m, 2H), 2.40 (m, 1H)_{∘}

### Step III: Preparation of compound 5

To a solution of (3,5-Difluoro-4-((6-(trifluoromethyl)pyridin-3-yl)oxy)phenyl)methanol **5c** (43 mg, 0.14 mmol) in dry *N,N*-dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound **5** (11 mg, 16.3 %) as a white solid.

¹H NMR (400 MHz, CDCl₃) δ 8.50 (m, 1H), 7.63 (m, 1H), 7.28 (m, *m* 1H), 7.15 (m, 2H), 5.45 (s, 2H), 4.33 - 4.27 (m, 2H), 4.20 (m, 2H), 3.78 (m, 2H), 3.46 - 3.39 (m, 2H). MS (ESI): m/z 482.9 [M+H]⁺_{∘}

### Example 6 Preparation of Compound 6

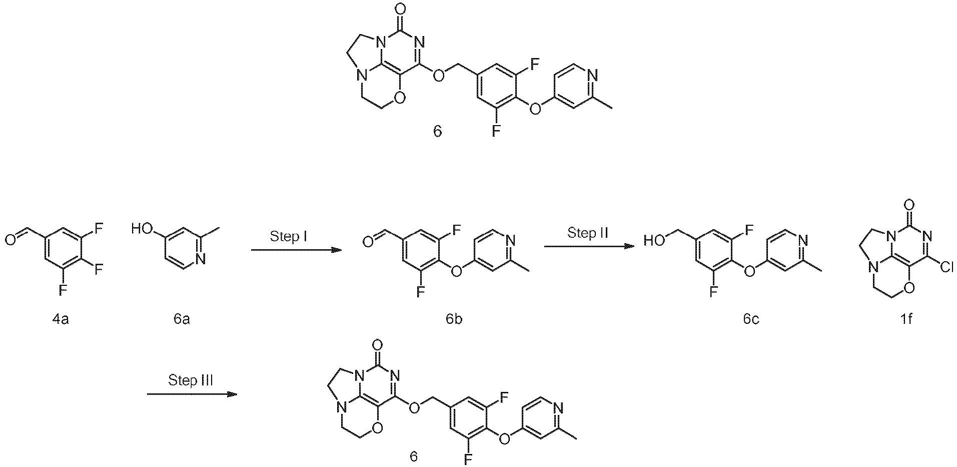

### Step I: Preparation of compound 6b

6-Methylpyridin-4-ol **6a** (0.5 g, 4.6 mmol), 3,4,5-trifluorobenzaldehyde **4a** (0.88 g, 5.5 mmol) and potassium carbonate (0.82 g, 5.95 mmol) were dissolved in N,N-dimethylformamide (DMF) (30 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **6b** (0.4 g, yield: 34.8%) as a yellow solid.

¹H NMR (400 MHz, CDCl₃) δ 9.94 (m, 1H), 8.39 (m, 1H), 7.63 - 7.55 (m, 2H), 6.72 - 6.64 (m, 2H), 2.50 (s, 3H).

### Step II: Preparation of compound 6c

At room temperature, 3,5-Difluoro-4-((2-methylpyridin-4-yl)oxy)benzaldehyde **6b** (0.4 g, 1.6 mmol) was dissolved in ethanol (50 mL), then NaBH₄ (71 mg, 1.87 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound 6c (0.34 g, yield: 85.7%) as a colorless oil.

¹H NMR (400 MHz, CDCl₃) δ 8.29 (m, 1H), 7.07 (m, 2H), 6.70 (m, 1H), 6.65 (m, 1H), 4.73 (s, 2H), 3.20 (m, 1H), 2.50 (s, 3H).

### Step III: Preparation of compound 6

To a solution of (3,5-Difluoro-4-((2-methylpyridin-4-yl)oxy)phenyl)methanol **6c** (35 mg, 0.14 mmol) in dry N,N-dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound 6 (6 mg, 10 %) as a white solid.

¹H NMR (400 MHz, CDCl₃) δ 8.35 (m, 1H), 7.12 (m, 2H), 6.67 (m, 2H), 5.44 (s, 2H), 4.33 - 4.27 (m, 2H), 4.19 (m, 2H), 3.78 (m, 2H), 3.45 - 3.38 (m, 2H), 2.50 (s, 3H). MS (ESI): m/z 429.0 [M+H]⁺_{∘}

### Example 7 Preparation of Compound 7

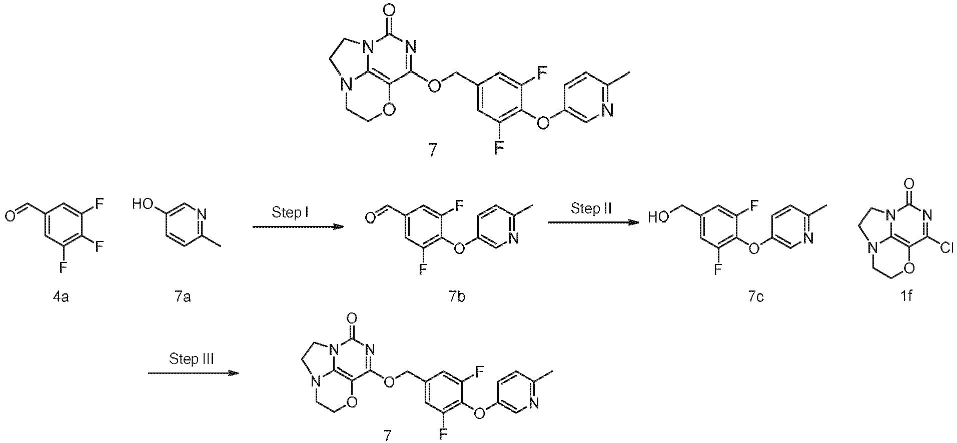

### Step I: Preparation of compound 7b

6-Methylpyridin-3-ol **7a** (0.57 g, 5.2 mmol), 3,4,5-trifluorobenzaldehyde **4a** (1 g, 6.2 mmol) and potassium carbonate (0.93 g, 6.76 mmol) were dissolved in N,N-dimethylformamide (DMF) (30 mL) at room temperature. The reaction mixture was stirred at 90°C for 1 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **7b** (0.91 g, yield: 70.2%) as a yellow solid.

¹H NMR (400 MHz, CDCl₃) δ 9.92 (s, 1H), 8.28 (s, 1H), 7.62 - 7.49 (m, 2H), 7.18 - 7.10 (m, 2H), 2.54 (s, 3H)_{∘}

### Step II: Preparation of compound 7c

At room temperature, 3,5-Difluoro-4-((6-methylpyridin-3-yl)oxy)benzaldehyde **7b** (0.91 g, 3.6 mmol) was dissolved in methanol (50 mL), then NaBH₄ (161 mg, 4.2 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound 7c (0.89 g, yield: 98.4%) as a colorless oil.

¹H NMR (400 MHz, CDCl₃) δ 8.20 (m, 1H), 7.16 - 6.98 (m, 4H), 4.69 (m, 2H), 2.88 (m, 1H), 2.50 (s, 3H)_{∘}

### Step III: Preparation of compound 7

To a solution of (3,5-Difluoro-4-((6-methylpyridin-3-yl)oxy)phenyl)methanol **7c** (35 mg, 0.14 mmol) in dry *N,N*-dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound **7** (12 mg, 20 %) as a white solid.

¹H NMR (400 MHz, CDCl₃) δ 8.27 (m, 1H), 7.14 - 7.02 (m, 4H), 5.42 (s, 2H), 4.32 - 4.25 (m, 2H), 4.19 (m, 2H), 3.77 (m, 2H), 3.45 - 3.36 (m, 2H), 2.51 (s, 3H). MS (ESI): m/z 429.0 [M+H]⁺_{∘}

### Example 8 Preparation of Compound 8

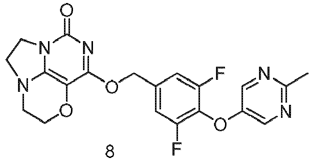

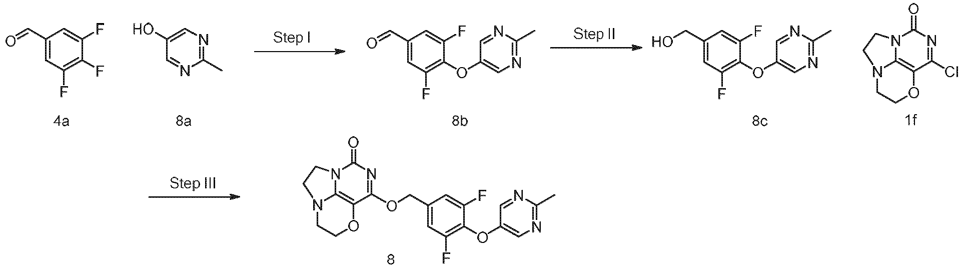

### Step I: Preparation of compound 8b

2-methylpyrimidin-5-ol **8a** (0.25 g, 2.3 mmol), 3,4,5-trifluorobenzaldehyde **4a** (0.44 g, 2.7 mmol) and potassium carbonate (0.41 g, 2.9 mmol) were dissolved in N,N-dimethylformamide (DMF) (30 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **8b** (0.24 g, yield: 41.7%) as a yellow solid.

¹H NMR (400 MHz, CDCl₃) δ 9.93 (s, 1H), 8.39 (s, 2H), 7.64 - 7.54 (m, 2H), 2.72 (s, 3H)_{∘}

### Step II: Preparation of compound 8c

At room temperature, 3,5-Difluoro-4-((2-methylpyrimidin-5-yl)oxy)benzaldehyde **8b** (0.24 g, 0.96 mmol) was dissolved in methanol (50 mL), then NaBH₄ (30 mg, 0.79 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound **8c** (0.17 g, yield: 70.2%) as a colorless oil.

¹H NMR (400 MHz, CDCl₃) δ 8.33 (s, 2H), 7.04 (m, 2H), 4.71 (m, 2H), 2.70 (s, 3H)_{∘}

### Step III: Preparation of compound 8

To a solution of (3,5-Difluoro-4-((2-methylpyrimidin-5-yl)oxy)phenyl)methanol **8c** (35 mg, 0.14 mmol) in dry *N,N*-dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound **8** (8 mg, 13.3 %) as a white solid.

¹H NMR (400 MHz, CDCl₃) δ 8.34 (s, 2H), 7.13 (m, 2H), 5.43 (s, 2H), 4.32 - 4.24 (m, 2H), 4.19 (m, 2H), 3.77 (m, 2H), 3.42 (m, 2H), 2.69 (s, 3H). MS (ESI): m/z 429.9 [M+H]⁺_{∘}

### Example 9 Preparation of Compound 9

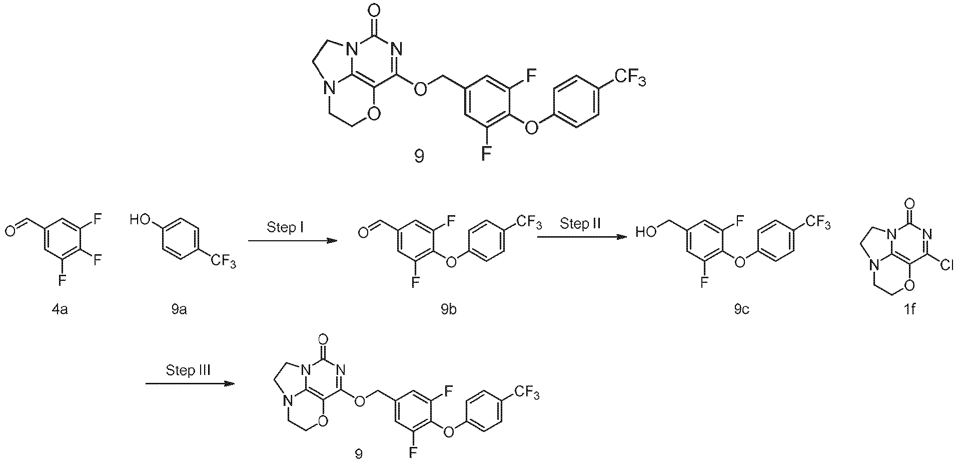

### Step I: Preparation of compound 9b

4-(Trifluoromethyl)phenol **9a** (0.84 g, 5.2 mmol), 3,4,5-trifluorobenzaldehyde **4a** (1 g, 6.2 mmol) and potassium carbonate (0.93 g, 6.76 mmol) were dissolved in N,N-dimethylformamide (DMF) (30 mL) at room temperature. The reaction mixture was stirred at 90°C for 1 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=5/1) afforded the title compound **9b** (1.33 g, yield: 84.6%) as a yellow solid.

¹H NMR (400 MHz, CDCl₃) δ 9.94 (m, 1H), 7.59 (m, 4H), 7.04 (m, 2H)_{∘}

### Step II: Preparation of compound 9c

At room temperature, 3,5-Difluoro-4-(4-(trifluoromethyl)phenoxy)benzaldehyde **9b** (1.33 g, 4.4 mmol) was dissolved in methanol (50 mL), then NaBH₄ (166 mg, 4.4 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=2/1) afforded the title compound **9c** (0.85 g, yield: 63.4%) as a colorless oil.

¹H NMR (400 MHz, CDCl₃) δ 7.57 (m, 2H), 7.09 - 7.00 (m, 4H), 4.72 (m, 2H), 2.03 (m, 1H)_{∘}

### Step III: Preparation of compound 9

To a solution of 3,5-Difluoro-4-(4-(trifluoromethyl)phenoxy)phenyl)methanol **9c** (43 mg, 0.14 mmol) in dry N,N-dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound **9** (9 mg, 13.4 %) as a white solid.

¹H NMR (400 MHz, CDCl₃) δ 7.56 (m, 2H), 7.16 - 7.09 (m, 2H), 7.00 (m, 2H), 5.44 (s, 2H), 4.30 (m, 2H), 4.20 (m, 2H), 3.78 (m, 2H), 3.46 - 3.35 (m, 2H). MS (ESI): m/z 481.9 [M+H]⁺_{∘}

### Example 10 Preparation of Compound 10

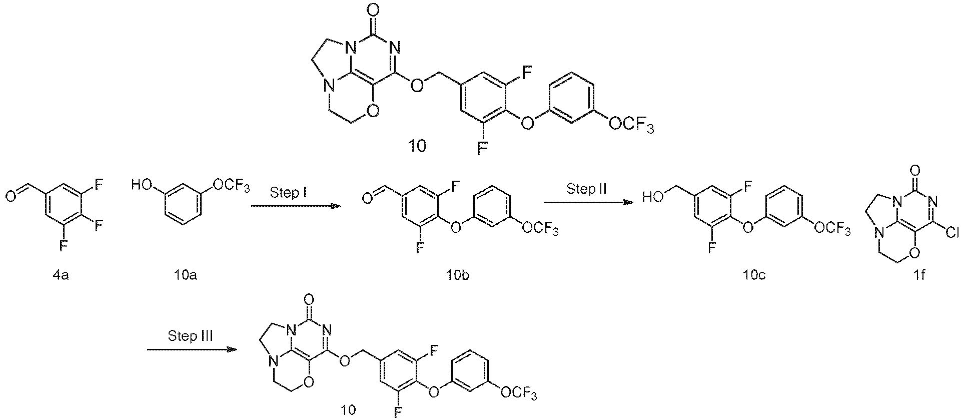

### Step I: Preparation of compound 10b

3-(Trifluoromethoxy)phenol **10a** (0.50 g, 2.8 mmol), 3,4,5-trifluorobenzaldehyde **4a** (0.5 g, 3.1 mmol) and potassium carbonate (0.5 g, 3.64 mmol) were dissolved in N,N-dimethylformamide (DMF) (30 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **10b** (0.73 g, yield: 81.9%) as a yellow solid.

¹H NMR (400 MHz, CDCl₃) δ 9.94 (s, 1H), 7.64 - 7.54 (m, 2H), 7.34 (m, 1H), 7.00 (m, 1H), 6.87 (m, 2H)_{∘}

### Step II: Preparation of compound 10c

At room temperature, 4-(3-(Trifluoromethoxy)phenoxy)-3,5-difluorobenzaldehyde **10b** (0.73 g, 2.3 mmol) was dissolved in methanol (50 mL), then NaBH₄ (86 mg, 2.26 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound **10c** (0.57 g, yield: 77.4%) as a colorless oil.

¹H NMR (400 MHz, CDCl₃) δ 7.30 (m, 1H), 7.06 (m, 2H), 6.94 (m, 1H), 6.85 (m, 1H), 6.81 (s, 1H), 4.72 (m, 2H), 1.94 (m, 1H)_{∘}

### Step III: Preparation of compound 10

To a solution of 4-(3-(Trifluoromethoxy)phenoxy)-3,5-difluorophenyl)methanol **10c** (48 mg, 0.15 mmol) in dry *N,N*-dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound **10** (18 mg, 25.8 %) as a white solid.

¹H NMR (400 MHz, CDCl₃) δ 7.30 (m, 1H), 7.11 (m, 2H), 6.93 (m, 1H), 6.83 (m, 2H), 5.44 (s, 2H), 4.34 - 4.27 (m, 2H), 4.20 (m, 2H), 3.78 (m, 2H), 3.46 - 3.38 (m, 2H). MS (ESI): m/z 497.9 [M+H]⁺_{∘}

### Example 11 Preparation of Compound 11

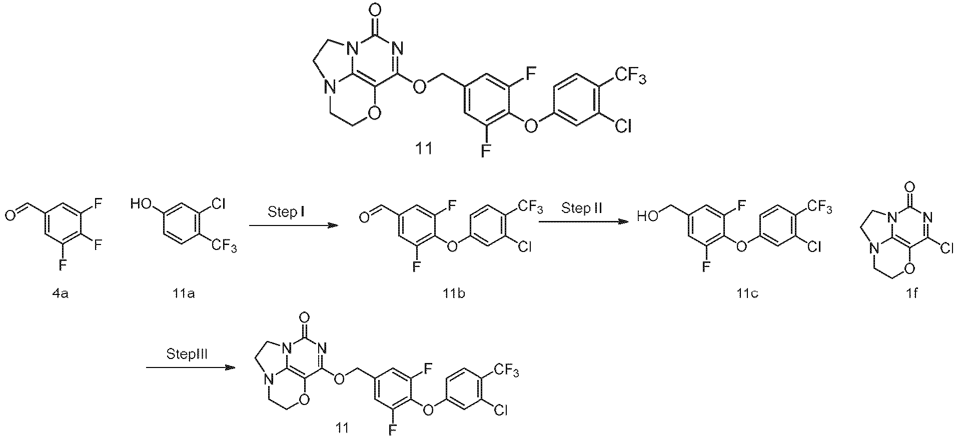

### Step I: Preparation of compound 11b

3-Chloro-4-(trifluoromethyl)phenol **11a** (0.25 g, 1.27 mmol), 3,4,5-trifluorobenzaldehyde **4a** (0.22 g, 1.4 mmol) and potassium carbonate (0.23 g, 1.65 mmol) were dissolved in N,N-dimethylformamide (DMF) (20 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound 11b (0.32 g, yield: 74.8%) as a yellow solid.

¹H NMR (400 MHz, CDCl₃) δ 9.95 (s, 1H), 7.69 - 7.56 (m, 3H), 7.10 (m, 1H), 6.92 (m, 1H)_{∘}

### Step II: Preparation of compound 11c

At room temperature, 4-(3-Chloro-4-(trifluoromethyl)phenoxy)-3,5-difluorobenzaldehyde **11b** (0.32 g, 0.95 mmol) was dissolved in methanol (50 mL), then NaBH₄ (36 mg, 0.95 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound **11c** (0.15 g, yield: 46.6%) as a white solid.

¹H NMR (400 MHz, CDCl₃) δ 7.62 (m, 1H), 7.13 - 7.00 (m, 3H), 6.90 (m, 1H), 4.74 (m, 2H), 1.88 (m, 1H)_{∘}

### Step III: Preparation of compound 11

To a solution of (4-(3-Chloro-4-(trifluoromethyl)phenoxy)-3,5-difluorophenyl)methanol **11c** (67 mg, 0.20 mmol) in dry *N,N-*dimethylformamide (5 mL) was added sodium hydride (60 % in mineral oil, 15 mg, 0.38 mmol) at 0 °C, and stirred at room temperature for 5 min. Then compound **1f** (40 mg, 0.19 mmol) was added, and stirred for 1 h, quenched with a small amount of water. Purification via silica gel column chromatography with an eluent system (dichloromethane/methanol=10/1) afforded the title compound 11 (21 mg, 21.4 %) as a white solid.

¹H NMR (400 MHz, CDCl₃) δ 7.62 (m, 1H), 7.14 (m, 2H), 7.06 (m, 1H), 6.89 (m, 1H), 5.45 (s, 2H), 4.34 - 4.26 (m, 2H), 4.21 (m, 2H), 3.78 (m, 2H), 3.52 - 3.37 (m, 2H). MS (ESI): m/z 515.9 [M+H]⁺_{∘}

### Example 12 Preparation of Compound 12

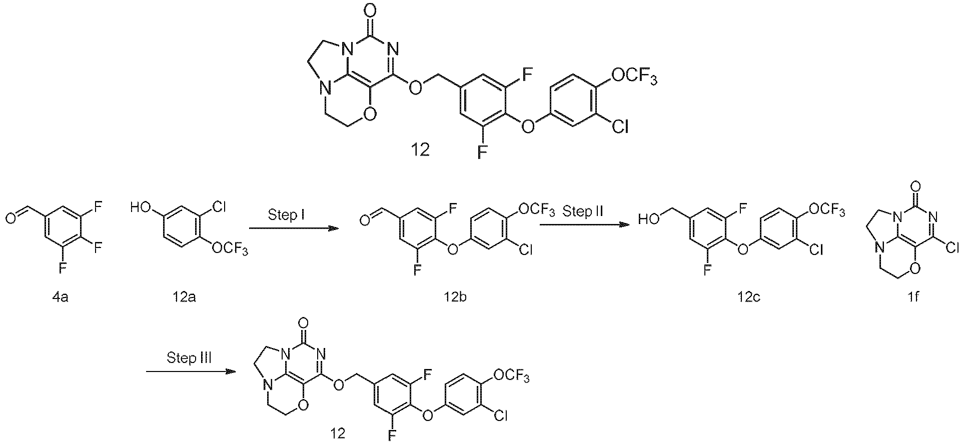

### Step I: Preparation of compound 12b

3-Chloro-4-(trifluoromethoxy)phenol **12a** (0.5 g, 2.4 mmol), 3,4,5-trifluorobenzaldehyde **4a** (0.41 g, 2.6 mmol) and potassium carbonate (0.42 g, 3.04 mmol) were dissolved in N,N-dimethylformamide (DMF) (20 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **12b** (0.62 g, yield: 73.2%) as a yellow solid.

¹H NMR (400 MHz, CDCl₃) δ 9.94 (s, 1H), 7.63 - 7.54 (m, 2H), 7.29 (m, 1H), 7.07 (m, 1H), 6.90 (m, 1H)_{∘}

### Step II: Preparation of compound 12c

At room temperature, 4-(3-Chloro-4-(trifluoromethoxy)phenoxy)-3,5-difluorobenzaldehyde **12b** (0.62 g, 1.8 mmol) was dissolved in methanol (50 mL), then NaBH₄ (62 mg, 1.63 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound **12c** (0.53 g, yield: 83.0%) as a colorless oil.

¹H NMR (400 MHz, CDCl₃) δ 7.25 (m, 1H), 7.06 (m, 2H), 7.01 (m, 1H), 6.87 (m, 1H), 4.72 (s, 2H), 2.04 (m, 1H)_{∘}

### Step III: Preparation of compound 12

To a solution of (4-(3-Chloro-4-(trifluoromethoxy)phenoxy)-3,5-difluorophenyl)methanol **12c** (67 mg, 0.19 mmol) in dry *N,N-*dimethylformamide (5 mL) was added sodium hydride (60 % in mineral oil, 15 mg, 0.38 mmol) at 0 °C, and stirred at room temperature for 5 min. Then compound **1f** (40 mg, 0.19 mmol) was added, and stirred for 1 h, quenched with a small amount of water. Purification via silica gel column chromatography with an eluent system (dichloromethane/methanol=10/1) afforded the title compound **12** (19 mg, 18.8 %) as a white solid.

¹H NMR (400 MHz, CDCl₃) δ 7.23 (m, 1H), 7.16 - 7.08 (m, 2H), 7.03 (m, 1H), 6.86 (m, 1H), 5.44 (s, 2H), 4.30 (m, 2H), 4.20 (m, 2H), 3.78 (m, 2H), 3.46 - 3.39 (m, 2H). MS (ESI): m/z 531.8 [M+H]⁺_{∘}

### Example 13 Preparation of Compound 13

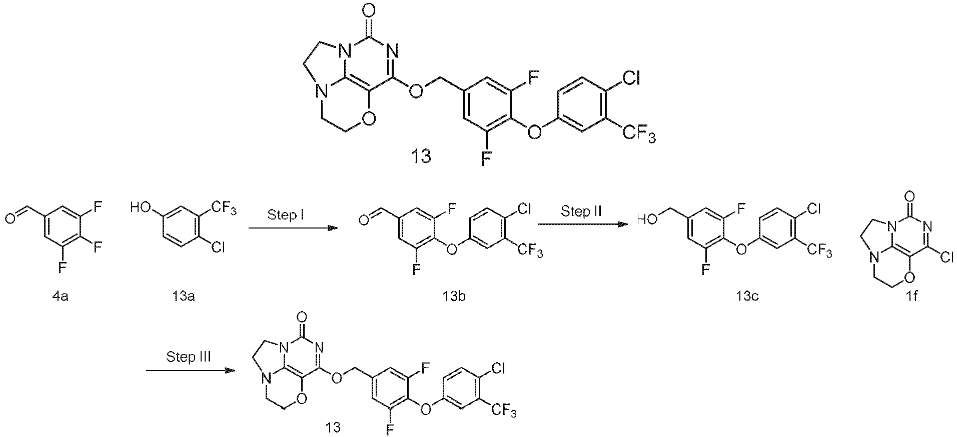

### Step I: Preparation of compound 13b

4-Chloro-3-(trifluoromethyl)phenol **13a** (0.5 g, 2.5 mmol), 3,4,5-trifluorobenzaldehyde **4a** (0.41 g, 2.8 mmol) and potassium carbonate (0.46 g, 3.3 mmol) were dissolved in N,N-dimethylformamide (DMF) (30 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **13b** (0.6 g, yield: 71.3%) as a yellow solid.

¹H NMR (400 MHz, CDCl₃) δ 9.94 (s, 1H), 7.64 - 7.55 (m, 2H), 7.45 (m, 1H), 7.31 (m, 1H), 7.05 (m, 1H)_{∘}

### Step II: Preparation of compound 13c

At room temperature, 4-(4-Chloro-3-(trifluoromethyl)phenoxy)-3,5-difluorobenzaldehyde 13b (0.6 g, 1.78 mmol) was dissolved in methanol (50 mL), then NaBH₄ (67 mg, 1.76 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound **13c** (0.28 g, yield: 46.4%) as a white solid.

¹H NMR (400 MHz, CDCl₃) δ 7.41 (m, 1H), 7.28 (m, 1H), 7.08 - 7.00 (m, 3H), 4.73 (m, 2H), 1.94 (m, 1H)_{∘}

### Step III: Preparation of compound 13

To a solution of (4-(4-Chloro-3-(trifluoromethyl)phenoxy)-3,5-difluorophenyl)methanol **13c** (64 mg, 0.19 mmol) in dry *N,N-*dimethylformamide (5 mL) was added sodium hydride (60 % in mineral oil, 15 mg, 0.38 mmol) at 0 °C, and stirred at room temperature for 5 min. Then compound **1f** (40 mg, 0.19 mmol) was added, and stirred for 1 h, quenched with a small amount of water. Purification via silica gel column chromatography with an eluent system (dichloromethane/methanol=10/1) afforded the title compound **13** (29 mg, 29.6 %) as a white solid.

¹H NMR (400 MHz, CDCl₃) δ 7.41 (m, 1H), 7.30 (m, 1H), 7.13 (m, 2H), 6.99 (m, 1H), 5.44 (s, 2H), 4.30 (m, 2H), 4.20 (m, 2H), 3.78 (m, 2H), 3.57 - 3.35 (m, 2H). MS (ESI): m/z 515.9 [M+H]⁺_{∘}

### Example 14 Preparation of Compound 14

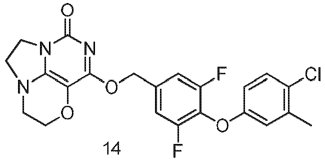

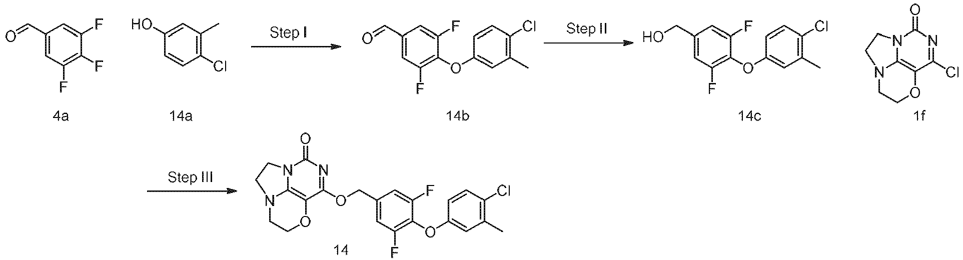

### Step I: Preparation of compound 14b

4-chloro-3-methylphenol **14a** (1 g, 7.0 mmol), 3,4,5-trifluorobenzaldehyde **4a** (1.2 g, 7.5 mmol) and potassium carbonate (1.3 g, 9.1 mmol) were dissolved in N,N-dimethylformamide (DMF) (30 mL) at room temperature. The reaction mixture was stirred at 90°C for 2 h, cooled to room temperature, followed by addition of ice water (100 mL), extracted with ethyl acetate (50 mL×3). The combined organic phases were washed with saturated aqueous sodium chloride, dried over anhydrous sodium sulfate, filtered to remove the desiccant. The filtrate was concentrated under reduced pressure. Purification via silica gel column chromatography with an eluent system (petroleum ether/ethyl acetate=10/1) afforded the title compound **14b** (1.2 g, yield: 60.6%) as a yellow solid.

¹H NMR (400 MHz, CDCl₃) δ 9.92 (s, 1H), 7.61 - 7.51 (m, 2H), 7.30 - 7.23 (m, 1H), 6.85 (m, 1H), 6.73 (m, 1H), 2.34 (s, 3H)_{∘}

### Step II: Preparation of compound 14c

At room temperature, 4-(4-Chloro-3-methylphenoxy)-3,5-difluorobenzaldehyde **14b** (1.2 g, 4.24 mmol) was dissolved in methanol (50 mL), then NaBH₄ (161 mg, 4.2 mmol) was added at 0 °C. The reaction mixture was stirred at room temperature for 0.5 h, concentrated under reduced pressure, followed by addition of water, extracted with ethyl acetate (100 mL×2). The combined organic phases were washed with saturated sodium chloride solution, dried over anhydrous sodium sulfate, filtered to remove desiccant. The filtrate was concentrated under reduced pressure. Purification via silica gel column chromatography with an eluent system (petroleum ether/ethyl acetate=4/1) afforded the title compound **14c** (0.89 g, yield: 73.7%) as a colorless oil.

### Step III: Preparation of compound 14

To a solution of (3,5-Difluoro-4-((2-(trifluoromethyl)pyridin-4-yl)oxy)phenyl)methanol **14c** (40 mg, 0.14 mmol) in dry *N,N-*dimethylformamide (5 mL) was added sodium hydride (60 % in mineral oil, 11 mg, 0.28 mmol) at 0 °C, and stirred at room temperature for 5 min. Then compound **1f** (30 mg, 0.14 mmol) was added, and stirred for 1 h, quenched with a small amount of water. Purification via silica gel column chromatography with an eluent system (dichloromethane/methanol=10/1) afforded the title compound **14** (16 mg, 24.7 %) as a white solid.

¹H NMR (400 MHz, CDCl₃) δ 7.23 (m, *m* 1H), 7.13 - 7.05 (m, 2H), 6.80 (m, 1H), 6.70 (m, 1H), 5.43 (s, 2H), 4.33 - 4.25 (m, 2H), 4.20 (m, 2H), 3.77 (m, 2H), 3.46 - 3.36 (m, 2H), 2.32 (s, 3H). MS (ESI): m/z 461.9 [M+H]⁺_{∘}

### Example 15 Preparation of Compound 15

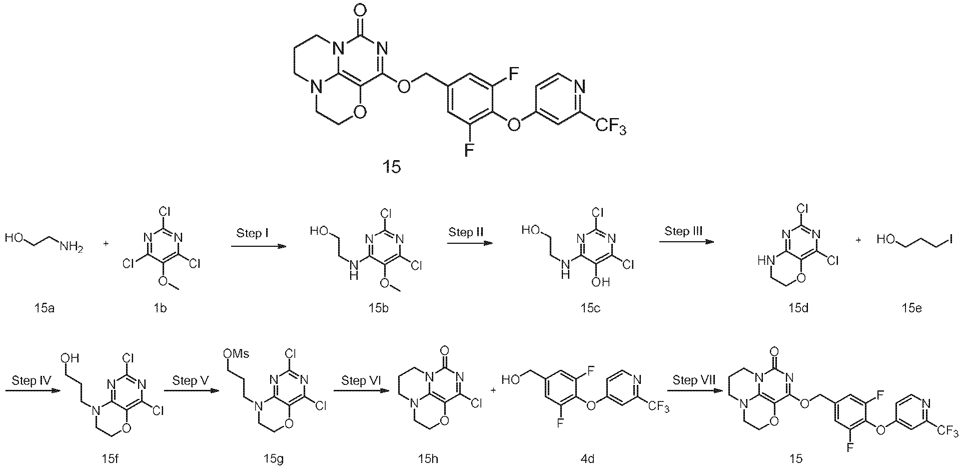

### Step I: Preparation of compound 15b

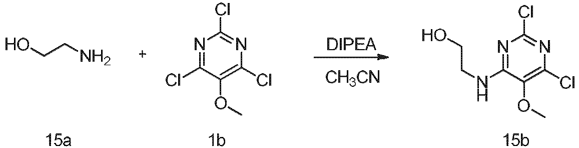

At room temperature, 2,4,6-trichloro-5-methoxypyrimidine **1b** (1.5 g, 7.03 mmol), ethanolamine **15a** (0.64 g, 10.48 mmol) and diisopropylethylamine (1.36 g, 10.54 mmol ) were dissolved in acetonitrile (70 mL), stirred and reacted for 3 h, concentrated under reduced pressure. Purification via silica gel column chromatography with an eluent system (dichloromethane/methanol=20/1) afforded the title compound **15b** (1.35 g, Yield: 80.6%) as an oil.

¹H NMR (400 MHz, DMSO) δ 7.99 (m, 1H), 4.77 (m, 1H), 3.73 (s, 3H), 3.52 (m, 2H), 3.39 (m, 2H).

### Step II: Preparation of compound 15c

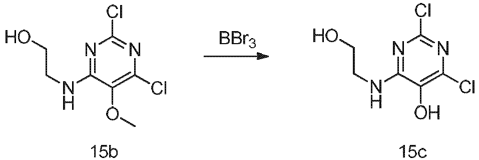

Under the protection of argon, **15b** (1.35 g, 5.7 mmol) was dissolved in anhydrous dichloromethane (50 mL), then boron tribromide (28.5 mL, 1M) was slowly added dropwise, and the reaction was stirred at 0°C for 4 h. The reaction was quenched by adding methanol, concentrated under reduced pressure. Purification via silica gel column chromatography with an eluent system (dichloromethane/methanol=10/1) afforded the title compound 15c (1.09 g, Yield: 85.4%) as a white solid.

### Step III: Preparation of compound 15d

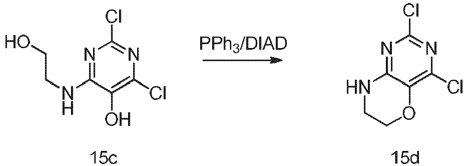

Under the protection of nitrogen, triphenylphosphine (3.8 g, 14.5 mmol) was dissolved in anhydrous THF (50 mL), then DIAD (2.87 mL, 14.6 mmol) was slowly added dropwise. The reaction mixture was stirred at 0°C for 15 min, and 15c was added dropwise (1.09 g, 4.87 mmol) in THF/DMF solution (30mL/5mL), then stirred at 0°C for 4 h, quenched by addition of water dropwise, extracted with ethyl acetate. The combined organic layers were dried over anhydrous sodium sulfate, concentrated under reduced pressure. Purification via silica gel column chromatography with an eluent system (dichloromethane/methanol=40/1) afforded the title compound **15d** (1.0 g, Yield: 99.6%) as a white solid.

¹H NMR (400 MHz, CDCl₃) δ 7.14 (s, 1H), 4.30 (m, 2H), 3.75 - 3.66 (m, 2H).

### Step IV: Preparation of compound 15f

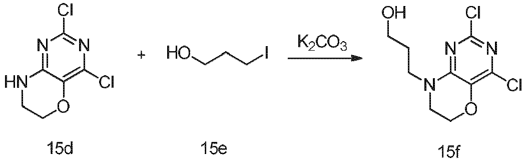

At room temperature, **15d** (1.0 g, 4.85 mmol) was dissolved in DMF (40 mL), followed by addition of potassium carbonate (0.92 g, 6.64 mmol) and 3-iodopropanol (1.23 g, 6.61 mmol). The reaction mixture was stirred at 80°C for 2 h, quenched by addition of water the reaction, extracted with ethyl acetate. The combined organic layers were dried over anhydrous sodium sulfate, concentrated under reduced pressure. Purification via silica gel column chromatography with an eluent system (dichloromethane/methanol=20/1) afforded the title compound **15f** (0.58 g, Yield: 45.3%) as a white solid.

¹H NMR (400 MHz, CDCl₃) δ 4.26 (m, 2H), 3.77 - 3.70 (m, 2H), 3.61 - 3.54 (m, 3H), 3.30 (m, 1H), 1.84 (m, 2H).

### Steps V and VI: Preparation of compound 15h

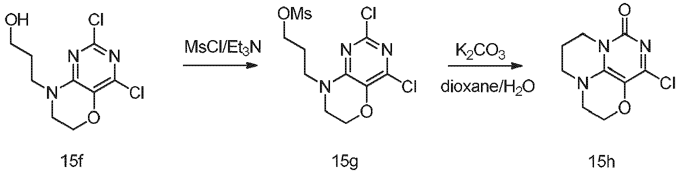

At room temperature, 15f (0.58 g, 2.2 mmol) and triethylamine (0.67 g, 6.6 mmol) were dissolved in dry dichloromethane (30 mL), then methanesulfonyl chloride (0.30 g, 2.6 mmol) was added dropwise at 0° C. The reaction mixture was stirred at 0°C for 1 h, concentrated under reduced pressure. The crude product from the previous step was dissolved in mixed solvent of dioxane/water (60 mL, 1/1), followed by addition of potassium carbonate (0.9 g, 6.6 mmol) at room temperature. The reaction mixture was stirred at 90°C overnight, concentrated under reduced pressure. Purification via silica gel column chromatography with an eluent system (dichloromethane/methanol=10/1) afforded the title compound **15h** (85 mg, Yield: 16.8%) as a white solid.

¹H NMR (400 MHz, CDCl₃) δ 4.19 - 4.14 (m, 2H), 3.97 - 3.90 (m, 2H), 3.56 (m, 2H), 3.44 (m, 2H), 2.14 (m, 2H).

### Step VII: Preparation of compound 15

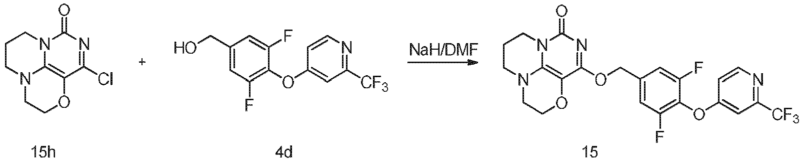

To a solution of (3,5-Difluoro-4-((2-(trifluoromethyl)pyridin-4-yl)oxy)phenyl)methanol **4d** (79 mg, 0.26 mmol) in dry N,N-dimethylformamide (5 mL) was added sodium hydride (60 % in mineral oil, 11 mg, 0.28 mmol) at 0 °C, and stirred at room temperature for 5 min. Then compound **15h** (40 mg, 0.18 mmol) was added, and stirred for 1 h, quenched with a small amount of water. Purification via silica gel column chromatography with an eluent system (dichloromethane/methanol=10/1) afforded the title compound **15** (12 mg, 13.4 %) as a white solid.

¹H NMR (400 MHz, CDCl₃) δ 8.61 (m, 1H), 7.20 (m, 3H), 6.99 (m, 1H), 5.47 (m, 2H), 4.19 (m, 2H), 4.05 - 3.97 (m, 2H), 3.58 - 3.50 (m, 2H), 3.40 (m, 2H), 2.17 (m, 2H). MS (ESI): m/z 497.1 [M+H]⁺_{∘}

### Example 16 Preparation of Compound 16

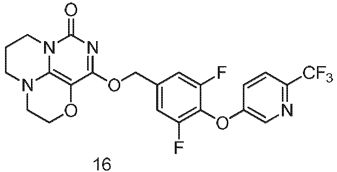

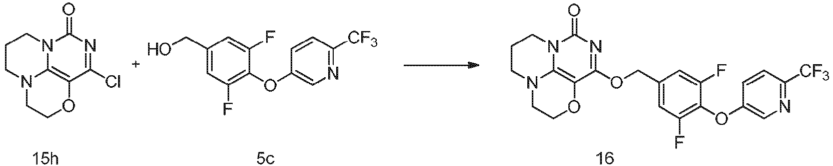

To a solution of (3,5-Difluoro-4-((6-(trifluoromethyl)pyridin-3-yl)oxy)phenyl)methanol 5c (61 mg, 0.2 mmol) in dry *N,N*-dimethylformamide (5 mL) was added sodium hydride (60 % in mineral oil, 8 mg, 0.2 mmol) at 0 °C, and stirred at room temperature for 5 min. Then compound **15h** (30 mg, 0.13 mmol) was added, and stirred for 1 h, quenched with a small amount of water. Purification via silica gel column chromatography with an eluent system (dichloromethane/methanol=10/1) afforded the title compound **16** (11 mg, 17.0 %) as a white solid.

¹H NMR (400 MHz, CDCl₃) δ 8.49 (m, 1H), 7.62 (m, 1H), 7.28 (m, *m* 1H), 7.17 (m, 2H), 5.43 (s, 2H), 4.17 (m, 2H), 4.03 - 3.95 (m, 2H), 3.55 - 3.49 (m, 2H), 3.37 (m, 2H), 2.15 (m, 2H). MS (ESI): m/z 497.1 [M+H]⁺_{∘}

### Example 17 Preparation of Compound 17

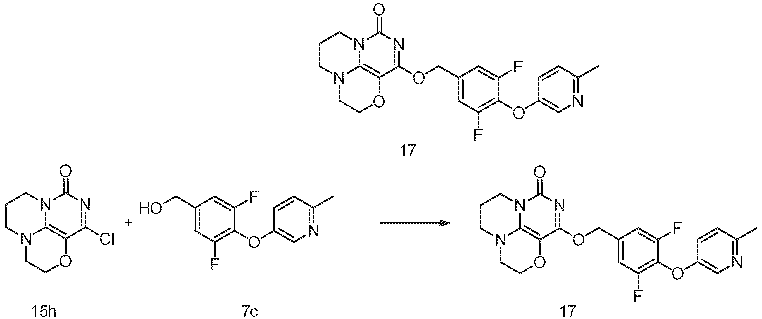

To a solution of (3,5-Difluoro-4-((6-methylpyridin-3-yl)oxy)phenyl)methanol **7c** (50 mg, 0.2 mmol) in dry *N,N*-dimethylformamide (5 mL) was added sodium hydride (60 % in mineral oil, 8 mg, 0.2 mmol) at 0 °C, and stirred at room temperature for 5 min. Then compound **15h** (30 mg, 0.13 mmol) was added, and stirred for 1 h, quenched with a small amount of water. Purification via silica gel column chromatography with an eluent system (dichloromethane/methanol=10/1) afforded the title compound **17** (18 mg, 31.3 %) as a white solid.

¹H NMR (400 MHz, CDCl₃) δ 8.26 (s, 1H), 7.09 (m, 4H), 5.42 (s, 2H), 4.16 (m, 2H), 3.99 (m, 2H), 3.51 (m, 2H), 3.36 (m, 2H), 2.51 (s, 3H), 2.14 (m, 2H). MS (ESI): m/z 443.1 [M+H]⁺_{∘}

### Example 18 Preparation of Compound 18

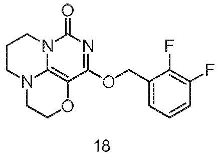

To a solution of (2,3-Difluorophenyl)methanol (38 mg, 0.26 mmol) in dry *N,N-*dimethylformamide (5 mL) was added sodium hydride (60 % in mineral oil, 11 mg, 0.28 mmol) at 0 °C, and stirred at room temperature for 5 min. Then compound **15h** (40 mg, 0.18 mmol) was added, and stirred for 1 h, quenched with a small amount of water. Purification via silica gel column chromatography with an eluent system (dichloromethane/methanol=10/1) afforded the title compound **18** (16 mg, 26.5 %) as a white solid.

¹H NMR (400 MHz, CDCl₃) δ 7.45 (m, 1H), 6.95 - 6.83 (m, 2H), 5.42 (s, 2H), 4.20 (m, 2H), 4.04 - 3.95 (m, 2H), 3.56 - 3.49 (m, 2H), 3.40 (m, 2H), 2.16 (m, 2H). MS (ESI): m/z 336.1 [M+H]⁺_{∘}

### Example 19 Preparation of Compound 19

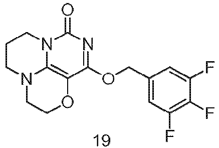

To a solution of (3,4,5-Trifluorophenyl)methanol (42 mg, 0.26 mmol) in dry *N,N-*dimethylformamide (5 mL) was added sodium hydride (60 % in mineral oil, 11 mg, 0.28 mmol) at 0 °C, and stirred at room temperature for 5 min. Then compound **15h** (40 mg, 0.18 mmol) was added, and stirred for 1 h, quenched with a small amount of water. Purification via silica gel column chromatography with an eluent system (dichloromethane/methanol=10/1) afforded the title compound **19** (15 mg, 23.6 %) as a white solid.

¹H NMR (400 MHz, CDCl₃) δ 7.33 (m, 2H), 5.43 (s, 2H), 4.20 (m, 2H), 4.06 - 3.96 (m, 2H), 3.57 - 3.49 (m, 2H), 3.41 (m, 2H), 2.18 (m, 2H). MS (ESI): m/z 354.1 [M+H]⁺_{∘}

### Biological evaluation

The biological activity of the compounds can be determined by using any suitable assay and tissue and in vivo models for determining the activity of the compounds as LpPLA2 inhibitors.

### (1) Recombinant human Lp-PLA2 (rhLp-PLA2) assay (also known as PED6 assay)

PED6 is a fluorescently labeled phospholipid that can be purchased directly from Invitogene or Molecular Probes. There is a fluorescence-quenching p-nitrophenyl group on the Sn3 position, and a Bodipy fluorescein (FL) group on the sn2 position. Once it is cleaved by the Lp-PLA2 enzyme, the FL group is released, resulting in enhanced fluorescence. However, Lp-PLA2 inhibitors can prevent this cleavage, so that no fluorescence enhancement is observed

Assay method: The compound to be tested (as shown in Table 1) was mixed with DMSO solution at a volume ratio of 1:3, diluted to prepare a source plate of a 384-well microplate. Then 0.01 µl of the compound was transferred via an ECHO liquid dispenser from the source plate to a 384-well Greiner 784076 plate, and 5 microliters of a buffer composed of 50 mM HEPES, pH7.4, 150 mM NaCl, 1 mM CHAPS (the buffer solution contains Recombinant human Lp-PLA2 enzyme at a concentration of 4 nM or 110 pM) was added to each well on the plate. The plate was centrifuged at 500 rpm for 10 seconds. After 30 minutes of pre-incubation, 5 microliters of the above buffer solution was added to a 384-well Greiner784076 plate, and the plate was centrifuged at 500 rpm for 10 seconds. After the plate was incubated at room temperature for 20 min in the dark, the fluorescence intensity was read at ex 480/em 540 with a ViewLux microplate imager, and the Excel's XL fitting model was used to performe curve and QC analysis to calculate pIC50. The results are listed in Table 1.

**Table 1**

| Compound No. | rhLp-PLA2 (pIC₅₀) |
|---|---|
| 1 | 7.8 |
| 2 | 8.0 |
| 3 | 8.5 |
| 4 | 9.9 |
| 5 | 9.7 |
| 6 | 9.8 |
| 7 | 9.6 |
| 8 | 9.0 |
| 9 | 9.8 |
| 10 | 9.7 |
| 11 | 10.0 |
| 12 | 10.1 |
| 13 | 9.8 |
| 14 | 9.7 |
| 15 | 10.2 |
| 16 | 10.1 |
| 17 | 10.3 |
| 18 | 8.0 |
| 19 | 8.4 |
| Positive Compound Rilapladib | 8.9 |

### (2) Human plasma Lp-PLA2 assay (also known as Thio-PAF assay)

The human plasma assay was conducted using the sulphatide analog of PAF (phosphatidylcholine), which is hydrolyzed to produce phospholipids containing free sulfhydryl groups, subjected to Michael addition with CPM to generate fluorescence-enhancing maleimide. Continuous quantitative analysis of thiol could be conducted by detecting the fluorescence intensity.

This assay can be used to detect the inhibitory activity of the Lp-PLA2 inhibitor on the Lp-PLA2 enzyme in human plasma.

Assay Method: The compound to be tested (as shown in Table 2) was mixed with a DMSO solution in a volume ratio of (1:3), and diluted to prepare a source plate of a 384-well microplate. Then 0.01 µl of the compound was transferred via an ECHO liquid dispenser from the source plate to a 384-well Greiner 784076 low-volume plate, and 8 µl of pre-aliquoted and frozen mixed human plasma was added. The plate was centrifuged at 500 rpm for 10 seconds. After a 30 min pre-incubation, 2 µl of a substrate solution, and containing 2.5 mM 2-thio-PAF (a solution in ethanol), 32 µM CPM (a solution in DMSO) and a buffer of 3.2 mM N-ethylmaleimide (NEM) (a buffer solution consisting of 50 mM HEPES, pH7.4, 150 mM NaCl, 1 mM CHAPS) was added by a BRAVO liquid handling station to a 384-well Greiner 784076 low-volume plate. After 2 min, the reaction was quenched with 5 µl of 5% trifluoroacetic acid. After the plate was incubated at room temperature for 40 min in a dark place, the fluorescence intensity was read at ex 380/ em 485 with an Envision microplate reader, and the XL fitting model in Excel was used to perform the curve analysis and QC analysis to calculate pIC50. The results are shown in Table 2.

**Table 2**

| Compound No. | Thio-PAF (pIC₅₀) |
|---|---|
| 1 | 6.5 |
| 2 | 6.8 |
| 3 | 7.3 |
| 4 | 7.9 |
| 5 | 7.7 |
| 6 | 7.8 |
| 7 | 7.7 |
| 8 | 7.4 |
| 9 | 8.0 |
| 10 | 7.8 |
| 11 | 8.0 |
| 12 | 7.8 |
| 13 | 7.9 |
| 14 | 7.8 |
| 15 | 8.1 |
| 16 | 8.0 |
| 17 | 8.1 |
| 18 | 6.9 |
| 19 | 7.2 |
| Positive compound Rilapladib | 7.8 |

## Claims

1. A compound of Formula (I) or a pharmaceutically acceptable salt thereof, 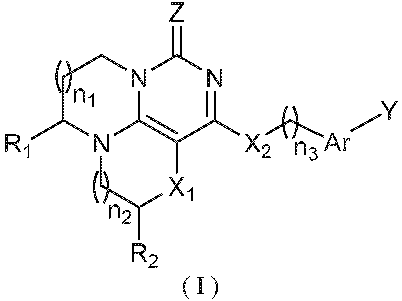 wherein
n₁, n₂, and n₃ are each independently 0, 1, or 2;
R₁ and R₂ are each independently selected from -H, hydroxyl, cyano, halogen, alkyl, deuterated alkyl, hydroxyalkyl, haloalkyl, cycloalkyl, alkoxy, haloalkoxy, deuterated alkoxy;
X₁ and X₂ are each independently selected from alkylene, -O-, -S-, or -NR';
R' is selected from -H, alkyl, deuterated alkyl, or cycloalkyl;
Ar is an arylene group or a heteroarylene group, wherein hydrogen atoms in the arylene or heteroarylene are optionally substituted by 0, 1 or more substituents, and the substituents are each independently selected from halogen, alkyl, deuteroalkyl, haloalkyl, alkoxy, deuteroalkoxy, haloalkoxy, hydroxy, hydroxyalkyl, cyano, amino, monoalkyl- or dialkyl-substituted amino, nitro, carboxyl, aldehyde, cycloalkyl, heterocyclyl, aryl, or heteroaryl;
Y is -H, halogen, alkyl, haloalkyl, haloalkoxy, cycloalkyl, alkoxy, deuterated alkyl, deuterated alkoxy, -OAr', -SAr', -NH-Ar', -NMe-Ar', -NR", or -R"'-Ar';
Ar' is selected from aryl or heteroaryl, wherein hydrogen atoms in the aryl or heteroaryl are optionally substituted with one or more substituents, the substituents are each independently selected from halogen, alkyl, deuterated alkyl, haloalkyl, alkoxy, deuterated alkoxy, hydroxy, hydroxyalkyl, haloalkoxy, cyano, amino, nitro, carboxyl,
aldehyde, cycloalkyl, heterocyclyl, aryl, or heteroaryl;
R" is alkyl;
R‴ is alkylene;
Z is O or S.

2. The compound or a salt thereof according to claim 1, wherein
halogens in the "halogen" "haloalkyl" and "haloalkoxy" are each independently selected from F, Cl, Br, or I;
and/or
alkyls in the "alkyl" "deuterated alkyl" "hydroxyalkyl" "haloalkyl" "haloalkoxy", "alkoxy" and "mono- or di-alkyl substituted amino" are each independently C₁-C₁₀ linear or branched alkyl; optionally each independently C₁-C₇ linear or branched alkyl; optionally each independently C₁-C₄ linear or branched alkyl; and optionally selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl;
and/or
"alkylenes" are each independently C₁-C₁₀ linear or branched alkylene; optionally each C₁-C₇ linear or branched alkylene; optionally each C₁-C₅ linear or branched alkylene; and optionally each selected from methylene, ethylene, n-propylene, iso-propylene, n-butylene, iso-butylene, tert-butylene, sec-butylene, n-pentylene, 1-methylbutylene, 2-methylbutylene, 3-methylbutylene, isopentylene, 1-ethylpropylene, neopentylene, n-hexylene, 1-methylpentylene, 2-methylpentylene, 3-methylpentylene, isohexylene, 1,1-dimethylbutylene, 2,2-dimethylbutylene, 3,3-dimethylbutylene, 1,2-dimethylbutylene, 1,3-dimethylbutylene, 2,3-dimethylbutylene, 2-ethylbutylene, n-heptylene, 2-methylhexylene, 3-methylhexylene, 2,2-dimethylpentylene, 3,3-dimethylpentylene, 2,3-dimethylpentylene, 2,4-dimethylpentylene, 3-ethylpentylene, or 2,2,3-trimethylbutylene;
and/or
"cycloalkyl" is C₃-C₁₀ monocyclic or bicyclic cycloalkyl, optionally C₃-C₇ monocyclic cycloalkyl, and optionally cyclopropyl, cyclobutyl, cyclopentyl, cyclohexyl, or cycloheptyl;
and/or
"heterocyclyl" is 3- to 10-membered non-aromatic heterocycle ring containing 1, 2, or 3 heteroatoms selected from N, O, and S; optionally 3- to 10-membered non-aromatic ring containing 1 or 2 heteroatoms selected from N and O; optionally 3- to 6-membered non-aromatic ring containing 1 or 2 heteroatoms selected from N and O; optionally 3- to 10-membered non-aromatic ring containing 1 or 2 heteroatoms selected from N and S; and optionally 3- to 6-membered non-aromatic ring containing 1 or 2 heteroatoms selected from N and S;
and/or
"aryl" is 6- to 10-membered aryl; optionally phenyl or naphthyl, and optionally phenyl, 1-naphthyl, or 2-naphthyl;
and/or
"arylene" is 6- to 10-membered arylene; and optionally phenylene or naphthylene;
and/or
"heteroaryl" is 5- to 10-membered heteroaryl ring containing 1-3 heteroatoms selected from N, O, and S; optionally 5- to 10-membered heteroaryl ring containing 1-2 heteroatoms selected from N, O, and S; optionally the heteroaryl ring is selected from pyridine ring, pyrrole ring, pyrazole ring, pyrimidine ring, pyrazine ring, pyridazine ring, thiophene ring, and furan ring; optionally selected from pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, pyrazin-3-yl, indolyl, isoindolyl, indazolyl, indolizinyl, purinyl, quinolizinyl, quinolinyl, isoquinolinyl, cinnolinyl, phthalazinyl, naphthyridinyl, quinazolinyl, quinoxalinyl, thieno[2,3-b] furanyl, furo[3,2-b]-pyranyl, pyrido[2,3-d]oxazinyl, pyrazolo[4,3-d]oxazolyl, imidazo[4,5-d]thiazolyl, pyrazino[2,3-d]pyridazinyl, imidazo[2,1-b]thiazolyl, imidazo[1,2-b][l,2,4]triazinyl, benzothienyl, benzoxazolyl, benzimidazolyl, benzothiazolyl, benzoxepinyl, benzoxazinyl, benzofuranyl, benzotriazolyl, pyrrolo[2,3-b]pyridyl, pyrrolo[3,2-c]pyridinyl, pyrrolo[3,2-b]pyridyl, imidazo[4,5-b]pyridyl, imidazo[4,5-c]pyridyl, pyrazolo[4,3-d]pyridyl, pyrazolo[4,3-c]pyridyl, pyrazolo[3,4-c]pyridinyl, pyrazolo[3,4-d]pyridyl, pyrazolo[3,4-b] pyridinyl, imidazo[1,2-a]pyridinyl, pyrazolo[1,5-a]pyridinyl, pyrrolo[1,2-b]pyridazinyl, imidazo[1,2-c]pyrimidinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazolo[2,3-b]pyrazinyl, or pyrimido[4,5-d]pyrimidinyl; and is optionally selected from pyridin-2-yl, pyridin-3-yl, pyridin-4-yl, pyrimidin-2-yl, pyrimidin-4-yl, or pyrimidin-5-yl;
and/or
"heteroarylene" is 5- to 10-membered heteroarylene ring containing 1-3 heteroatoms selected from N, O, and S; optionally 5- to 10-membered heteroaromatic ring containing 1-2 heteroatoms selected from N, O, and S; and optionally the heteroarylene ring is selected from pyridine ring, pyrrole ring, pyrazole ring, pyrimidine ring, pyrazine ring, pyridazine ring, thiophene ring, furan ring.

3. The compound or a salt thereof according to claim 1 or 2, wherein
n₁ is 0 or 1; and/or n₂ is 1; and/or n₃ is 1;
and/or
R₁ and R₂ are each independently selected from -H, fluorine, chlorine, bromine, hydroxyl, cyano, C₁-C₇ alkyl (such as methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), C₁-C₃ deuteroalkyl (such as -CD₃, -C₂D₅, or -C₃D₇), C₁-C₃ deuteroalkoxyl (such as -OCD₃, - OC₂D₅, or -OC₃D₇), haloalkyl, haloalkoxyl, cyclopropanyl, cyclobutanyl, cyclopentanyl; optionally, R₁ is -H or -CH₃; and optionally, R₁ is-H, R₂ is-H;
and/or
X₁ and X₂ are each independently selected from C₁-C₇ alkylene (optionally, -CH₂-, ethylene, n-propylene, isopropylene, n-butylene, or isobutylene), -O-, -S-, or - NR'-; optionally, X₁ is -CH₂-, or -O-; optionally, X₁ is -O-; optionally, X₂ is -O-; optionally, R' is selected from -H, C₁-C₇ alkyl (optionally, methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), deuterated alkyl (optionally, -CD₃, -C₂D₅, or - C₃D₇), or C₃-C₆ cycloalkyl (optionally, cyclopropanyl, cyclobutanyl, cyclopentanyl, or cyclohexanyl);
and/or
Ar is phenylene or pyridyl, wherein hydrogen atoms in the phenylene or pyridyl are optionally substituted with 0, 1, 2, or 3 substituents, the substituents are each independently selected from F, Cl, Br, I, -CN, -Me, -C₂H₅, cyclopropyl, -CD₃, - OMe, -OCD₃, -CF₃, or -OCF₃; optionally, Ar is phenylene, wherein the hydrogen atom in the phenylene is optionally substituted by 2 substituents, and the substituent is F;
and/or
Y is -H, -F, -Cl, -Br, methyl, ethyl, n-propyl, isopropyl, -CD₃, -CF₃, -CH₂CF₃, -OCF₃, -OCHF₂, -OCH₂F, cyclopropyl, cyclobutyl, cyclopentyl, -OCH₃, -OCD₃, - OC₂H₅, -OC₃H₇, or -OAr';
optionally, Y is H, halogen, or -OAr'; and optionally, Y is H, -F, or -OAr';
Ar' is selected from phenyl, pyridyl, pyrimidyl, pyrrolyl, pyrazolyl, thienyl or quinolinyl, and the phenyl, pyridyl, pyrimidyl, pyrrolyl, pyrazolyl, thienyl or quinoline optionally, Ar' is selected from phenyl, pyridyl, pyrimidyl, pyrrolyl, pyrazolyl, thienyl or quinolinyl, wherein hydrogen atoms in the phenyl, pyridyl, pyrimidyl, pyrrolyl, pyrazolyl, thienyl or quinolinyl ring are each independently optionally substituted with 1, 2, or 3 substituents, the substituents are each independently selected from F, Cl, Br, - CN, C₁-C₇ alkyl (optionally, methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), -CD₃, C₁-C₆ haloalkyl, -OCH₃, -OCD₃, -OC₂H₇, -OC₃H₇, C₁-C₆ haloalkoxyl, or C₃-C₆ cycloalkyl (optionally, cyclopropanyl, cyclobutanyl, cyclopentanyl, or cyclohexanyl);
optionally, Ar' is selected from phenyl, pyridin-3-yl, pyridin-4-yl, or pyrimidin-5-yl, and is optionally substituted with 1 or 2 substituents, the substituents are selected from F, Cl, -CH₃, -CF₃, or-OCF₃; and/or Z is O.

4. The compound of Formula (I) or a pharmaceutically acceptable salt thereof according to any one of claims 1-3, wherein the compound of Formula (I) is selected from the following compounds:

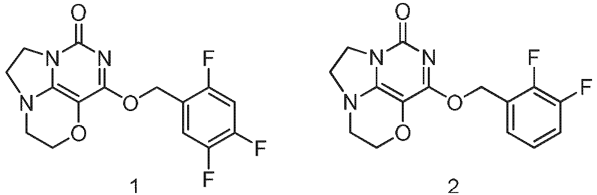

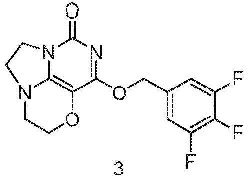

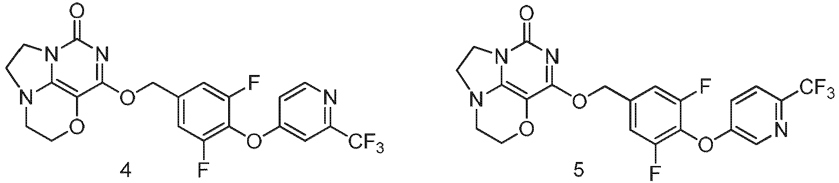

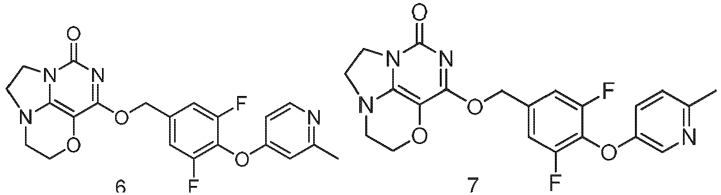

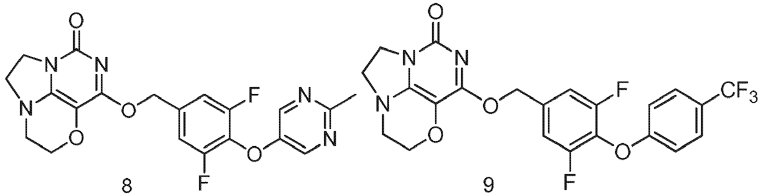

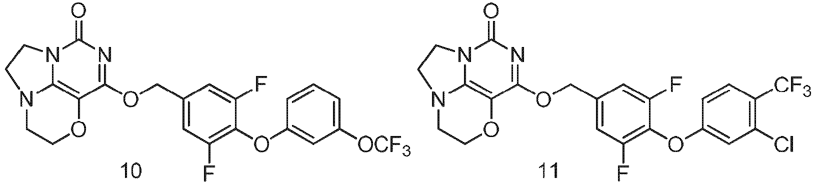

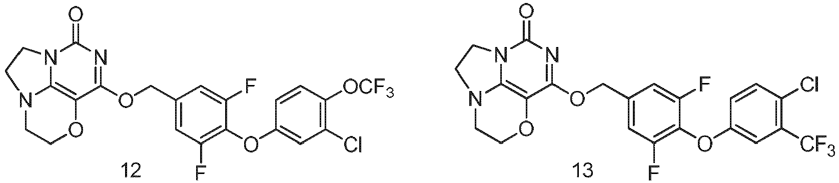

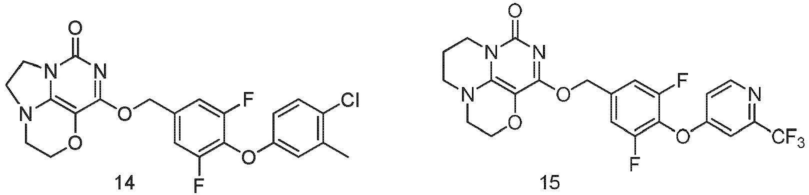

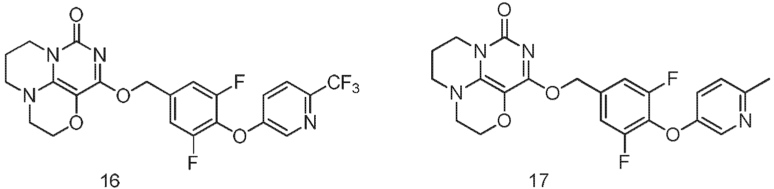

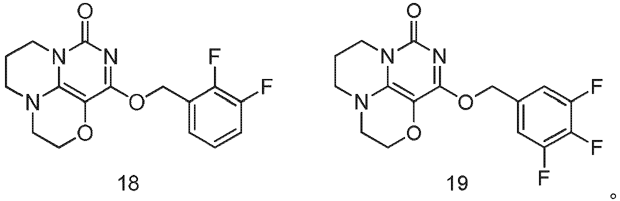

5. The compound of Formula (I) or a pharmaceutically acceptable salt thereof according to any one of claims 1-4, wherein the pharmaceutically acceptable salt includes an anionic salt or cationic salt of the compound of Formula (I);
wherein the pharmaceutically acceptable salt includes alkali metal salt, alkaline earth metal salt, or ammonium salt of the compound of Formula (I); optionally, the alkali metal includes sodium, potassium, lithium, or cesium, and the alkaline earth metal includes magnesium, calcium, or strontium;
or the pharmaceutically acceptable salt includes salt formed by the compound of Formula **(I)** and an organic base;
optionally, the organic base includes trialkylamine, pyridine, quinoline, piperidine, imidazole, picoline, dimethylaminopyridine, dimethylaniline, N-alkylmorpholine, 1,5-diazabicyclo[4.3.0]nonene-5, 1,8-diazabicyclo[5.4.0]undecene-7, 1,4-diazabicyclo[2.2.2] octane; optionally, the trialkylamine includes trimethylamine, triethylamine, or N-ethyldiisopropylamine; and optionally, the N-alkyl morpholine includes N-methylmorpholine;
or the pharmaceutically acceptable salt includes salt formed by the compound of Formula **(I)** and an acid;
optionally, the acid includes inorganic acid, or organic acid; optionally, the inorganic acid includes hydrochloric acid, hydrobromic acid, hydroiodic acid, sulfuric acid, nitric acid, phosphoric acid, or carbonic acid; optionally, the organic acid includes formic acid, acetic acid, propionic acid, oxalic acid, malonic acid, succinic acid, fumaric acid, maleic acid, lactic acid, malic acid, citric acid, citric acid, tartaric acid, carbonic acid, picric acid, methanesulfonic acid, ethanesulfonic acid, p-toluenesulfonic acid, glutamic acid, or pamoic acid.

6. A preparation method of the compound of Formula (I) or its tautomer, mesomer, racemate, enantiomer, diastereoisomer, its mixture form or its pharmaceutically acceptable salt according to any one of claims 1-5, comprising the following synthetic route: 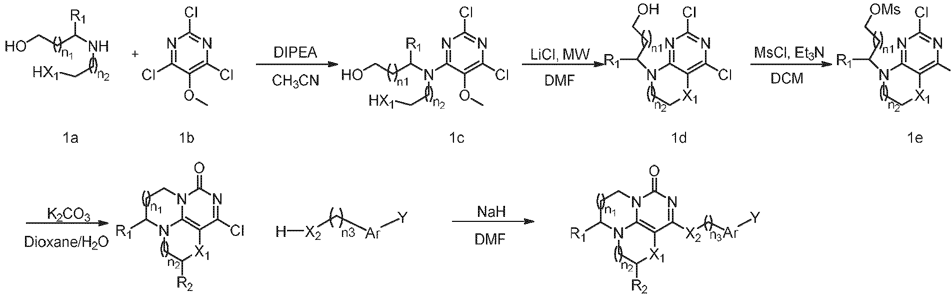 in each of the formulas in the preparation method described above, n₁, n₂, n₃, R₁, R₂, X₁, X₂, Ar, and Y are defined as any of claims 1-5.

7. A pharmaceutical composition comprising the compound of formula (I) according to any one of claims 1-5 or its tautomer, mesoform, racemate, enantiomer, diastereomer or mixtures thereof, or pharmaceutically acceptable salts thereof, and optionally a pharmaceutically acceptable carrier.

8. The pharmaceutical composition according to claim 7, comprising the compound of formula (I) or its tautomer, mesomer, racemate, enantiomer, diastereomer , or a mixture thereof, or one or more of a pharmaceutically acceptable salt thereof and optionally a pharmaceutically acceptable carrier;
wherein the oral formulation includes solid or liquid formulation;
optionally, the solid formulation includes tablet, powder, granule, or capsule;
optionally, the liquid formulation includes aqueous or oily suspension, or syrup;
optionally, the parenteral formulation includes solution for injection, or aqueous or oily suspension.

9. Use of the compound of Formula (I) or a pharmaceutically acceptable salt thereof according to any one of claims 1-5, or the pharmaceutical composition according to claim 7 or 8, in the preparation of an Lp-PLA2 inhibitor.

10. Use of the compound of Formula (I) or a pharmaceutically acceptable salt thereof according to any one of claims 1-5, or the pharmaceutical composition according to claim 7 or 8, in the preparation of a medicament for treatment of neurodegeneration-related diseases;
optionally, the neurodegeneration-related diseases include Alzheimer's disease (AD), glaucoma, and age-related macular degeneration (AMD).

11. Use of the compound of Formula (I) or a pharmaceutically acceptable salt thereof according to any one of claims 1-5, or the pharmaceutical composition according to claim 7 or 8, in the preparation of a medicament for the treatment of cardiovascular diseases, diabetic macular edema (DME), or prostate diseases;
optionally, the cardiovascular diseases include atherosclerosis.

## Patentansprüche

1. Verbindung der Formel (I) oder ein pharmazeutisch unbedenkliches Salz davon, 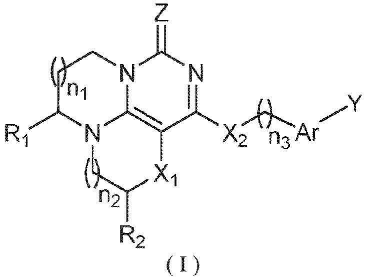 wobei:
n₁, n₂ und n₃ jeweils unabhängig 0, 1 oder 2 sind;
R₁ und R₂ jeweils unabhängig aus -H, Hydroxyl, Cyano, Halogen, Alkyl, deuteriertem Alkyl, Hydroxyalkyl, Haloalkyl, Cycloalkyl, Alkoxy, Haloalkoxy, deuteriertem Alkoxy ausgewählt sind;
X₁ und X₂ jeweils unabhängig aus Alkylen, -O-, -S- oder -NR'- ausgewählt sind;
R' aus -H, Alkyl, deuteriertem Alkyl oder Cycloalkyl ausgewählt ist;
Ar eine Arylengruppe oder eine Heteroarylengruppe ist, wobei Wasserstoffatome im Arylen oder Heteroarylen wahlweise durch 0, 1 oder mehr Substituenten substituiert sind, und die Substituenten jeweils unabhängig aus Halogen, Alkyl, Deuteroalkyl, Haloalkyl, Alkoxy, Deuteroalkoxy, Haloxyalkoxy, Hydroxy, Hydroxyalkyl, Cyano, Amino, monoalkyl- oder dialkylsubstituiertem Amino, Nitro, Carboxyl, Aldehyd, Cycloalkyl, Heterocyclyl, Aryl oder Heteroaryl ausgewählt sind;
Y -H, Halogen, Alkyl, Haloalkyl, Haloalkoxy, Cycloalkyl, Alkoxy, deuteriertes Alkyl, deuteriertes Alkoxy, -OAr', -SAr', -NH-Ar', -NMe-Ar', -NR" oder -R‴-Ar' ist;
Ar' aus Aryl oder Heteroaryl ausgewählt ist, wobei Wasserstoffatome im Aryl oder Heteroaryl wahlweise durch einen oder mehrere Substituenten substituiert sind, wobei die Substituenten jeweils unabhängig aus Halogen, Alkyl, deuteriertem Alkyl, Haloalkyl, Alkoxy, deuteriertem Alkoxy, Hydroxy, Hydroxyalkyl, Haloalkoxy, Cyano, Amino, Nitro, Carboxyl, Aldehyd, Cycloalkyl, Heterocyclyl, Aryl oder Heteroaryl ausgewählt sind;
R" Alkyl ist;
R‴ Alkylen ist;
Z O oder S ist.

2. Verbindung oder ein Salz davon nach Anspruch 1, wobei
Halogene in "Halogen", "Haloalkyl" und "Haloalkoxy" jeweils unabhängig aus F, Cl, Br oder I ausgewählt sind;
und/oder Alkyle in "Alkyl", "deuteriertem Alkyl" "Hydroxyalkyl" "Haloalkyl" "Haloalkoxy", "Alkoxy" und "mono- oder di-alkyl-substituierten Amino" jeweils unabhängig lineares oder verzweigtes C₁-C₁₀-Alkyl sind; wahlweise jeweils unabhängig lineares oder verzweigtes C₁-C₇-Alkyl; wahlweise jeweils unabhängig lineares oder verzweigtes C₁-C₄-Alkyl; und wahlweise aus Methyl, Ethyl, n-Propyl, Isopropyl, n-Butyl, Isobutyl, tert-Butyl, sec-Butyl, n-Pentyl, 1-Methylbutyl, 2-Methylbutyl, 3-Methylbutyl, Isopentyl, 1-Ethylpropyl, Neopentyl, n-Hexyl, 1-Methylpentyl, 2-Methylpentyl, 3-Methylpentyl, Isohexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 2-Ethylbutyl, n-Heptyl, 2-Methylhexyl, 3-Methylhexyl, 2,2-Dimethylpentyl, 3,3-Dimethylpentyl, 2,3-Dimethylpentyl, 2,4-Dimethylpentyl, 3-Ethylpentyl oder 2,2,3-Trimethylbutyl ausgewählt sind;
und/oder "Alkylene" jeweils unabhängig voneinander lineares oder verzweigtes C₁-C₁₀-Alkylen sind; wahlweise jeweils lineares oder verzweigtes Alkylen C₁-C₇; wahlweise jeweils lineares oder verzweigtes C₁-C₅-Alkylen sind; und wahlweise jeweils aus Methylen, Ethylen, n-Propylen, iso-Propylen, n-Butylen, iso-Butylen, tert-Butylen, sec-Butylen, n-Pentylen, 1-Methylbutylen, 2-Methylbutylen, 3-Methylbutylen, Isopentylen, 1-Ethylpropylen, Neopentylen, n-Hexylen, 1-Methylpentylen, 2-Methylpentylen, 3-Methylpentylen, Isohexylen, 1,1-Dimethylbutylen, 2,2-Dimethylbutylen, 3,3-Dimethylbutylen, 1,2-Dimethylbutylen, 1,3-Dimethylbutylen, 2,3-Dimethylbutylen, 2-Ethylbutylen, n-Heptylen, 2-Methylhexylen, 3-Methylhexylen, 2,2-Dimethylpentylen, 3,3-Dimethylpentylen, 2,3-Dimethylpentylen, 2,4-Dimethylpentylen, 3-Ethylpentylen oder 2,2,3-Trimethylbutylen ausgewählt sind;
und/oder "Cycloalkyl" monocyclisches oder bicyclisches C₃-C₁₀-Cycloalkyl, wahlweise monocyclisches C₃-C₇-Cycloalkyl und wahlweise Cyclopropyl, Cyclobutyl, Cyclopentyl, Cyclohexyl oder Cycloheptyl ist;
und/oder "Heterocyclyl" ein 3- bis 10-gliedriger nichtaromatischer Heterocyclusring, der 1, 2 oder 3 Heteroatome enthält, die aus N, O und S ausgewählt sind; wahlweise ein 3- bis 10-gliedriger nichtaromatischer Ring, der 1 oder 2 Heteroatome enthält, die aus N und O ausgewählt sind; wahlweise ein 3- bis 6-gliedriger nichtaromatischer Ring, der 1 oder 2 Heteroatome enthält, die aus N und ausgewählt sind; wahlweise ein 3- bis 10-gliedriger nichtaromatischer Ring, der 1 oder 2 Heteroatome enthält, die aus N und S ausgewählt sind; und wahlweise ein 3- bis 10-gliedriger nichtaromatischer Ring, der 1 oder 2 Heteroatome enthält, die aus N und S ausgewählt sind;
und/oder "Aryl" 6- bis 10-gliedriges Aryl ist; wahlweise Phenyl oder Naphthyl und wahlweise Phenyl, 1-Naphthyl oder 2-Naphthyl;
und/oder "Arylen" 6- bis 10-gliedriges Arylen ist; und wahlweise Phenylen oder Naphthylen;
und/oder "Heteroaryl" ein 5- bis 10-gliedriger Heteroarylring ist, der 1 bis 3 Heteroatome enthält, die aus N, O und S ausgewählt sind; wahlweise ein 5- bis 10-gliedriger Heteroarylring, der 1 bis 2 Heteroatome enthält, die aus N, O und S ausgewählt sind; wahlweise der Heteroarylring aus Pyridinring, Pyrrolring, Pyrazolring, Pyrimidinring, Pyrazinring, Pyridazinring, Thiophenring und Furanring ausgewählt ist; wahlweise ausgewählt aus Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, Pyrazin-3-yl, Indolyl, Isoindolyl, Indazolyl, Indolizinyl, Purinyl, Chinolizinyl, Chinolinyl, Isochinolinyl, Cinnolinyl, Phthalazinyl, Naphthyridinyl, Chinazolinyl, Chinoxalinyl, Thieno[2,3-b]furanyl, Furo[3,2-b]-pyranyl, Pyrido[2,3-d]oxazinyl, Pyrazolo[4,3-d]oxazolyl, Imidazo[4,5-d]thiazolyl, Pyrazino[2,3-d]pyridazinyl, Imidazo[2,1-b]thiazolyl, Imidazo[1,2-b][1,2,4]triazinyl, Benzothienyl, Benzoxazolyl, Benzimidazolyl, Benzothiazolyl, Benzoxepinyl, Benzoxazinyl, Benzofuranyl, Benzotriazolyl, Pyrrolo[2,3-b]pyridyl, Pyrrolo[3,2-c]pyridinyl, Pyrrolo[3,2-b]pyridyl, Imidazo[4,5-b]pyridyl, Imidazo[4,5-c]pyridyl, Pyrazolo[4,3-d]pyridyl, Pyrazolo[4,3-c]pyridyl, Pyrazolo[3,4-c]pyridinyl, Pyrazolo[3,4-d]pyridyl, Pyrazolo[3,4-b] Pyridinyl, Imidazo[1,2-a]pyridinyl, Pyrazolo[1,5-a]pyridinyl, Pyrrolo[1,2-b]pyridazinyl, Imidazo[1,2-c]pyrimidinyl, Pyrido[3,2-d]pyrimidinyl, Pyrido[4,3-d]pyrimidinyl, Pyrido[3,4-d]pyrimidinyl, Pyrido[2,3-d]pyrimidinyl, Pyrido[2,3-b]pyrazinyl, Pyrido[3,4-b]pyrazinyl, Pyrimido[5,4-d]pyrimidinyl, Pyrazolo[2,3-b]pyrazinyl oder Pyrimido[4,5-d]pyrimidinyl; und wahlweise aus Pyridin-2-yl, Pyridin-3-yl, Pyridin-4-yl, Pyrimidin-2-yl, Pyrimidin-4-yl oder Pyrimidin-5-yl ausgewählt ist;
und/oder "Heteroarylen" ein 5- bis 10-gliedriger Heteroarylenring ist, der 1 bis 3 Heteroatome enthält, die aus N, O und S ausgewählt sind; wahlweise ein 5- bis 10-gliedriger heteroaromatischer Ring ist, der 1 bis 2 Heteroatome enthält, die aus N, O und S ausgewählt sind; und wahlweise der Heteroarylenring aus Pyridinring, Pyrrolring, Pyrazolring, Pyrimidinring, Pyrazinring, Pyridazinring, Thiophenring, Furanring ausgewählt ist.

3. Verbindung oder ein Salz davon nach Anspruch 1 oder 2, wobei
n₁ 0 oder 1 ist; und/oder n₂ 1 ist; und/oder n₃ 1 ist;
und/oder R₁ und R₂ jeweils unabhängig aus -H, Fluor, Chlor, Brom, Hydroxyl, Cyano, C₁-C₇-Alkyl (wie etwa Methyl, Ethyl, n-Propyl, Isopropyl, n-Butyl, Isobutyl, tert-Butyl, sec-Butyl, n-Pentyl, 1-Methylbutyl, 2-Methylbutyl, 3-Methylbutyl, Isopentyl, 1-Ethylpropyl, Neopentyl, n-Hexyl, 1-Methylpentyl, 2-Methylpentyl, 3-Methylpentyl, Isohexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 2-Ethylbutyl, n-Heptyl, 2-Methylhexyl, 3-Methylhexyl, 2,2-Dimethylpentyl, 3,3-Dimethylpentyl, 2,3-Dimethylpentyl, 2,4-Dimethylpentyl, 3-Ethylpentyl oder 2,2,3-trimethylbutyl), C₁-C₃-Deuteroalkyl (wie etwa -CD₃, -C₂D₅ oder -C₃D₇), C₁-C₃-Deuteroalkoxyl (wie etwa - OCD₃, -OC₂D₅ oder -OC₃D₇), Haloalkyl, Haloalkoxyl, Cyclopropanyl, Cyclobutanyl, Cyclopentanyl ausgewählt sind; wahlweise R₁ -H oder -CH₃ ist; und wahlweise R₁ - H ist, R₂ -H ist;
und/oder X₁ und X₂ jeweils unabhängig aus C₁-C₇-Alkylen (wahlweise -CH₂-, Ethylen, n-Propylen, Isopropylen, n-Butylen oder Isobutylen), -O-, -S- oder - NR'-ausgewählt sind; wahlweise X₁ -CH₂- oder -O- ist; wahlweise X₁ -O-; ist wahlweise X₂ -O- ist;
wahlweise R' aus -H, C₁-C₇-Alkyl (wahlweise Methyl, Ethyl, n-Propyl, Isopropyl, n-Butyl, Isobutyl, tert-Butyl, sec-Butyl, n-Pentyl, 1-Methylbutyl, 2-Methylbutyl, 3-Methylbutyl, Isopentyl, 1-Ethylpropyl, Neopentyl, n-Hexyl, 1-Methylpentyl, 2-Methylpentyl, 3-Methylpentyl, Isohexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 2-Ethylbutyl, n-Heptyl, 2-Methylhexyl, 3-Methylhexyl, 2,2-Dimethylpentyl, 3,3-Dimethylpentyl, 2,3-Dimethylpentyl, 2,4-Dimethylpentyl, 3-Ethylpentyl oder 2,2,3-Trimethylbutyl), deuteriertem Alkyl (wahlweise -CD₃, -C₂D₅ oder - C₃D₇) oder C₃-C6-Cycloalkyl (wahlweise Cyclopropanyl, Cyclobutanyl, Cyclopentanyl oder Cyclohexanyl) ausgewählt ist;
wahlweise Ar Phenylen oder Pyridyl ist, wobei Wasserstoffatome im Phenylen oder Pyridyl wahlweise mit 0, 1, 2 oder 3 Substituenten substituiert sind, die Substituenten jeweils unabhängig aus F, Cl, Br, I, -CN, -Me, C₂H₅, Cyclopropyl, - CD₃, -OMe, -OCD₃, -CF₃ oder -OCF₃ ausgewählt sind; wahlweise Ar Phenylen ist, wobei das Wasserstoffatom in dem Phenylen wahlweise durch 2 Substituenten substituiert sind und der Substituent F ist;
und/oder Y -H, -F, -Cl, -Br, Methyl, Ethyl, n-Propyl, Isopropyl, -CD₃, -CF₃, -CH₂CF₃, -OCF₃, -OCHF₂, -OCH₂F, Cyclopropyl, Cyclobutyl, Cyclopentyl, -OCH₃, -OCD₃, - OC₂H₅, -OC₃H₇ oder -OAr' ist;
wahlweise Y H, Halogen oder -OAr' ist; und wahlweise Y H, -F oder -OAr' ist;
Ar' aus Phenyl, Pyridyl, Pyrimidyl, Pyrrolyl, Pyrazolyl, Thienyl oder Chinolinyl und dem Phenyl, Pyridyl, Pyrimidyl, Pyrrolyl, Pyrazolyl, Thienyl oder Chinolin ausgewählt ist, wahlweise
Ar' aus Phenyl, Pyridyl, Pyrimidyl, Pyrrolyl, Pyrazolyl, Thienyl or Chinolinyl ausgewählt Ist, wobei Wasserstoffatome In dem Phenyl-, Pyridyl-, Pyrimidyl-, Pyrrolyl-, Pyrazolyl-, Thienyl- oder Chinolinylring jeweils unabhängig mit 1, 2 oder 3 Substituenten substituiert sind, die Substituenten jeweils unabhängig aus F, Cl, Br- , -CN, C₁-C₇-Alkyl (wahlweise Methyl, Ethyl, n-Propyl, Isopropyl, n-Butyl, Isobutyl, tert-Butyl, sec-Butyl, n-Pentyl, 1-Methylbutyl, 2-Methylbutyl, 3-Methylbutyl, Isopentyl, 1-Ethylpropyl, Neopentyl, n-Hexyl, 1-Methylpentyl, 2-Methylpentyl, 3-Methylpentyl, Isohexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 2-Ethylbutyl, n-Heptyl, 2-Methylhexyl, 3-Methylhexyl, 2,2-Dimethylpentyl, 3,3-Dimethylpentyl, 2,3-Dimethylpentyl, 2,4-Dimethylpentyl, 3-Ethylpentyl oder 2,2,3-Trimethylbutyl), -CD₃, C₁-C₆-Haloalkyl, -OCH₃, -OCD₃, -OC₂H₇, -OC₃H₇, C₁-C₆-Haloalkoxyl oder C₃-C₆-Cycloalkyl (wahlweise Cyclopropanyl, Cyclobutanyl, Cyclopentanyl oder Cyclohexanyl) ausgewählt sind; wahlweise Ar' aus Phenyl, Pyridin-3-yl, Pyridin-4-yl oder Pyrimidin-5-yl ausgewählt ist und wahlweise mit 1 oder 2 Substituenten substituiert ist, wobei die Substituenten aus F, Cl, -CH₃, -CF₃ oder-OCF₃ ausgewählt sind;
und/oder Z O ist.

4. Verbindung der Formel (I) oder ein pharmazeutisch unbedenkliches Salz davon nach einem der Ansprüche 1 bis 3, wobei die Verbindung der Formel (I) aus den folgenden Verbindungen ausgewählt ist:

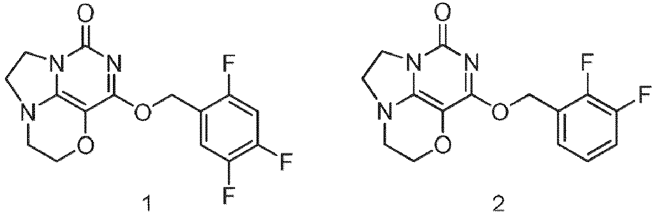

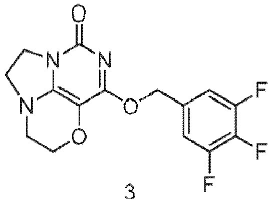

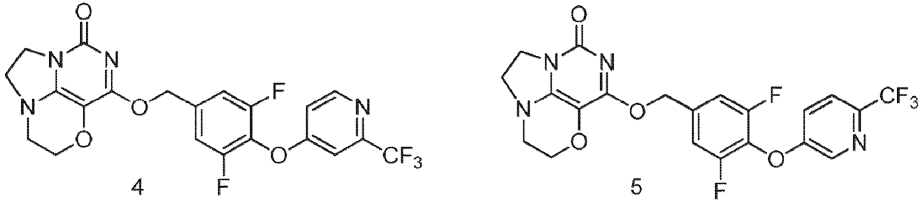

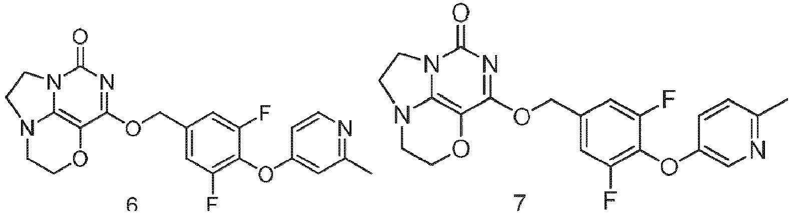

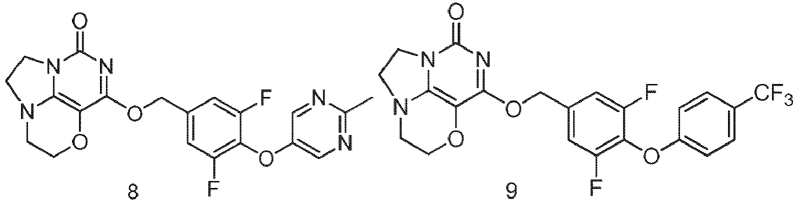

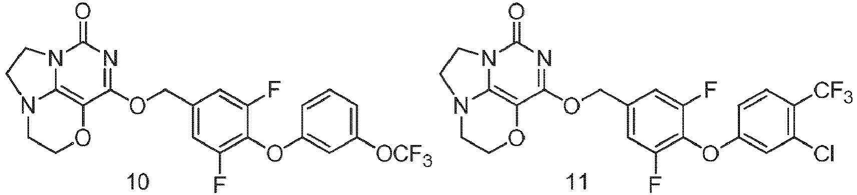

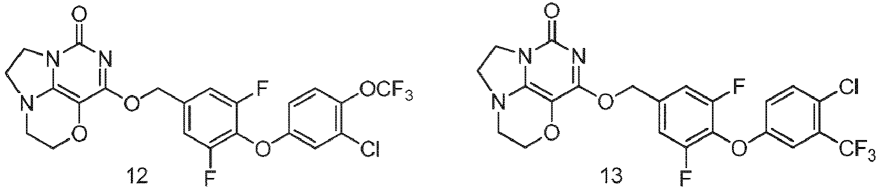

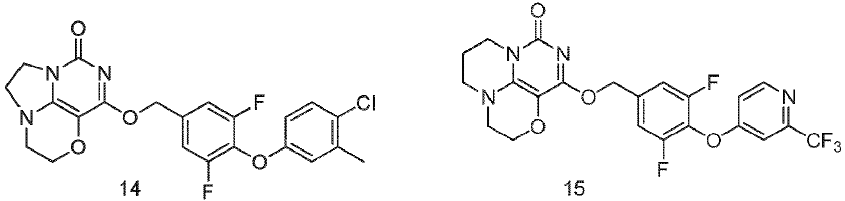

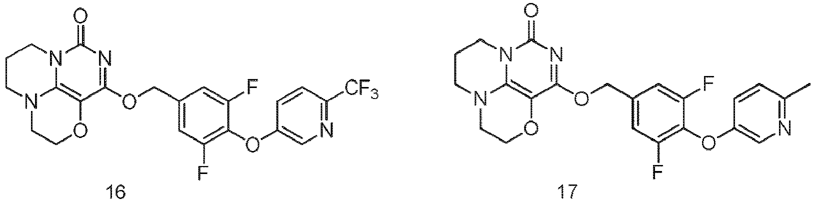

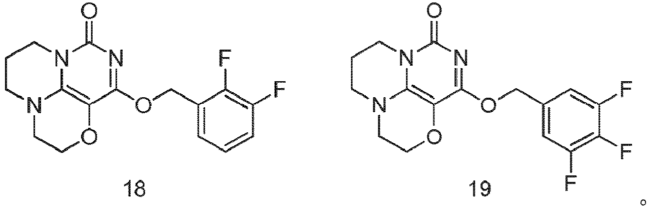

5. Verbindung der Formel (I) oder ein pharmazeutisch unbedenkliches Salz davon nach einem der Ansprüche 1 bis 4, wobei das pharmazeutisch unbedenkliche Salz ein anionisches Salz oder kationisches Salz der Verbindung der Formel (I) beinhaltet;
wobei das pharmazeutisch unbedenkliche Salz Alkalimetallsalz, Erdalkalimetallsalz oder Ammoniumsalz der Verbindung der Formel (I) beinhaltet; wahlweise das Alkalimetall Natrium, Kalium, Lithium oder Cäsium beinhaltet und das Erdalkalimetall Magnesium, Calcium oder Strontium beinhaltet;
oder das pharmazeutisch unbedenkliche Salz beinhaltet, das durch die Verbindung der Formel (I) und eine organische Base gebildet wird;
wahlweise die organische Base Trialkylamin, Pyridin, Chinolin, Piperidin, Imidazol, Picolin, Dimethylaminopyridin, Dimethylanilin, N-Alkylmorpholin, 1,5-Diazabicyclo[4,3.0]non-5-en, 1,8-Diazabicyclo[5,4.0]undec-7-en, 1,4-Diazabicyclo[2,2.2]octan beinhaltet; wahlweise das Trialkylamin Trimethylamin, Triethylamin oder N-Ethyldiisopropylamin beinhaltet; und optional das N-Alkylmorpholin N-Methylmorpholin beinhaltet;
oder das pharmazeutisch unbedenkliche Salz Salz, das durch die Verbindung der Formel (I) und eine Säure gebildet wird;
wahlweise die Säure anorganische Säure oder organische Säure beinhaltet;
wahlweise die anorganische Säure Salzsäure, Bromwasserstoffsäure, lodwasserstoffsäure, Schwefelsäure, Salpetersäure, Phosphorsäure oder Kohlensäure beinhaltet; wahlweise die organische Säure Ameisensäure, Essigsäure, Propionsäure, Oxalsäure, Malonsäure, Bernsteinsäure, Fumarsäure, Maleinsäure, Milchsäure, Apfelsäure, Zitronensäure, Zitronensäure, Weinsäure, Kohlensäure, Pikrinsäure, Methansulfonsäure, Ethansulfonsäure, p-Toluolsulfonsäure, Glutaminsäure oder Embonsäure beinhaltet.

6. Verfahren zum Herstellen der Verbindung der Formel (I) oder eines Tautomers, Mesomers, Racemats, Enantiomers, Diastereoisomers, einer Mischform oder eines pharmazeutisch unbedenklichen Salzes davon nach einem der Ansprüche 1 bis 5, umfassend den folgenden Syntheseweg: 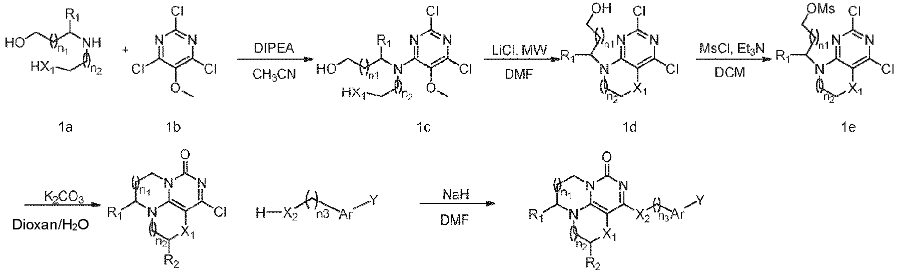 wobei in jeder der Formeln des oben beschriebenen Herstellungsverfahrens n₁, n₂, n₃, R₁, R₂, X₁, X₂, Ar und Y wie nach einem der Ansprüche 1 bis 5 definiert sind.

7. Pharmazeutische Zusammensetzung, umfassend die Verbindung der Formel (I) nach einem der Ansprüche 1-5 oder ein Tautomer, Mesoform, Racemat, Enantiomer, Diastereomer oder Mischungen davon oder pharmazeutisch unbedenkliche Salze davon und wahlweise einen pharmazeutisch unbedenklichen Träger.

8. Pharmazeutische Zusammensetzung nach Anspruch 7, umfassend die Verbindung der Formel (I) oder ein Tautomer, Mesoform, Racemat, Enantiomer, Diastereomer oder Mischung davon oder ein oder mehrere eines pharmazeutisch unbedenkliche Salze davon und wahlweise einen pharmazeutisch unbedenklichen Träger;
wobei die orale Formulierung feste oder flüssige Formulierungen beinhaltet;
wahlweise die feste Formulierung Tablette, Pulver, Granulat oder Kapsel beinhaltet;
wahlweise die flüssige Formulierung wässrige oder ölige Suspension oder Sirup beinhaltet;
wahlweise die parenterale Formulierung Injektionslösung oder wässrige oder ölige Suspension beinhaltet.

9. Verwendung der Verbindung der Formel (I) oder eines pharmazeutisch unbedenklichen Salzes davon nach einem der Ansprüche 1 bis 5 oder der pharmazeutischen Zusammensetzung nach Anspruch 7 oder 8 bei der Herstellung eines Lp-PLA2-Inhibitors.

10. Verwendung der Verbindung der Formel (I) oder eines pharmazeutisch unbedenklichen Salzes davon nach einem der Ansprüche 1 bis 5 oder der pharmazeutischen Zusammensetzung nach Anspruch 7 oder 8 bei der Herstellung eines Medikaments zur Behandlung neurodegenerationsbedingter Erkrankungen;
wobei wahlweise die neurodegenerationsbedingten Erkrankungen Alzheimer-Krankheit (AD), Glaukom und altersabhängige Makuladegeneration (AMD) sind.

11. Verwendung der Verbindung der Formel (I) oder eines pharmazeutisch unbedenklichen Salzes davon nach einem der Ansprüche 1 bis 5 oder der pharmazeutischen Zusammensetzung nach Anspruch 7 oder 8 bei der Herstellung eines Medikaments zur Behandlung von Herz-Kreislauf-Erkrankungen, diabetischem Makulaödem (DME) oder Prostatakrankheiten;
wahlweise wobei die Herz-Kreislauf-Erkrankungne Atherosklerose beinhalten.

## Revendications

1. Composé de formule (I) ou un sel pharmaceutiquement acceptable de celui-ci, 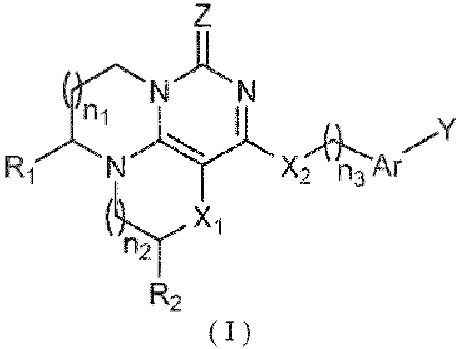 dans lequel
n₁, n₂ et n₃ sont chacun indépendamment 0, 1 ou 2 ;
R₁ et R₂ sont chacun indépendamment choisis parmi -H, hydroxyle, cyano, halogène, alkyle, alkyle deutéré, hydroxyalkyle, halogénoalkyle, cycloalkyle, alcoxy, halogénoalcoxy, alcoxy deutéré ;
X₁ et X₂ sont chacun indépendamment choisis parmi alkylène, -O-, -S- ou -NR'- ;
R' est choisi parmi -H, alkyle, alkyle deutéré ou cycloalkyle ;
Ar est un groupe arylène ou un groupe hétéroarylène, dans lequel les atomes d'hydrogène de l'arylène ou de l'hétéroarylène sont éventuellement substitués par 0, 1 ou plusieurs substituants, et les substituants sont chacun étant indépendamment choisis parmi halogène, alkyle, deutéroalkyle, halogénoalkyle, alcoxy, deutéroalcoxy, halogénoalcoxy, hydroxy, hydroxyalkyle, cyano, amino, amino à substitution monoalkyle ou dialkyle, nitro, carboxyle, aldéhyde, cycloalkyle, hétérocyclyle, aryle ou hétéroaryle ;
Y représente -H, halogène, alkyle, halogénoalkyle, halogénoalcoxy, cycloalkyle, alcoxy, alkyle deutéré, alcoxy deutéré, -OAr', -SAr', -NH-Ar', -NMe-Ar', -NR" ou -R"'-Ar' ;
Ar' est choisi parmi aryle ou hétéroaryle, dans lequel les atomes d'hydrogène de l'aryle ou de l'hétéroaryle sont éventuellement substitués par un ou plusieurs substituants, les substituants sont chacun indépendamment choisis parmi halogène, alkyle, alkyle deutéré, halogénoalkyle, alcoxy, alcoxy deutéré, hydroxy, hydroxyalkyle, halogénoalcoxy, cyano, amino, nitro, carboxyle, aldéhyde, cycloalkyle, hétérocyclyle, aryle ou hétéroaryle ;
R" est un groupe alkyle ;
R‴ est un groupe alkylène ;
Z est O ou S.

2. Composé ou sel de celui-ci selon la revendication 1, dans lequel
les halogènes dans « halogène », « halogénoalkyle » et « halogénoalcoxy » sont chacun indépendamment choisis parmi F, Cl, Br ou I ; et/ou
les alkyles dans « alkyle », « alkyle deutéré », « hydroxyalkyle », « halogénoalkyle », « halogénoalcoxy », « alcoxy » et « amino à substitution mono- ou di-alkyle » sont chacun indépendamment un groupe alkyle linéaire ou ramifié en C₁ à C₁₀ ; éventuellement chacun indépendamment un groupe alkyle linéaire ou ramifié en C₁ à C₇ ; éventuellement chacun indépendamment un groupe alkyle linéaire ou ramifié en C₁ à C₄ ;et éventuellement choisi parmi méthyle, éthyle, n-propyle, isopropyle, n-butyle, isobutyle, tert-butyle, sec-butyle, n-pentyle, 1-méthylbutyle, 2-méthylbutyle, 3-méthylbutyle, isopentyle, 1-éthylpropyle, néopentyle, n-hexyle, 1-méthylpentyle, 2-méthylpentyle, 3-méthylpentyle, isohexyle, 1,1-diméthylbutyle, 2,2-diméthylbutyle, 3,3-diméthylbutyle, 1,2-diméthylbutyle, 1,3-diméthylbutyle, 2,3-diméthylbutyle, 2-éthylbutyle, n-heptyle, 2-méthylhexyle, 3-méthylhexyle, 2,2-diméthylpentyle, 3,3-diméthylpentyle, 2,3-diméthylpentyle, 2,4-diméthylpentyle, 3-éthylpentyle ou 2,2,3-triméthylbutyle ;
et/ou
les « alkylènes » sont chacun indépendamment un groupe alkylène linéaire ou ramifié en C₁ à C₁₀ ; éventuellement chacun un groupe alkylène linéaire ou ramifié en C₁ à C₇ ; éventuellement chacun un groupe alkylène linéaire ou ramifié en C₁ à C₅ ; et éventuellement chacun choisi parmi méthylène, éthylène, n-propylène, isopropylène, n-butylène, isobutylène, tert-butylène, sec-butylène, n-pentylène, 1-méthylbutylène, 2-méthylbutylène, 3-méthylbutylène, isopentylène, 1-éthylpropylène, néopentylène, n-hexylène, 1-méthylpentylène, 2-méthylpentylène, 3-méthylpentylène, isohexylène, 1,1-diméthylbutylène, 2,2-diméthylbutylène, 3,3-diméthylbutylène, 1,2-diméthylbutylène, 1,3-diméthylbutylène, 2,3-diméthylbutylène, 2-éthylbutylène, n-heptylène, 2-méthylhexylène, 3-méthylhexylène, 2,2-diméthylpentylène, 3,3-diméthylpentylène, 2,3-diméthylpentylène, 2,4-diméthylpentylène, 3-éthylpentylène ou 2,2,3-triméthylbutylène ;
et/ou
« cycloalkyle » est un groupe cycloalkyle monocyclique ou bicyclique en C₃ à C₁₀, éventuellement un groupe cycloalkyle monocyclique en C₃ à C₇, et éventuellement un groupe cyclopropyle, cyclobutyle, cyclopentyle, cyclohexyle ou cycloheptyle ;
et/ou
« hétérocyclyle » est un cycle hétérocyclique non aromatique de 3 à 10 chaînons contenant 1, 2 ou 3 hétéroatomes choisis parmi N, O et S ; éventuellement un cycle non aromatique de 3 à 10 chaînons contenant 1 ou 2 hétéroatomes choisis parmi N et O ; éventuellement un cycle non aromatique de 3 à 6 chaînons contenant 1 ou 2 hétéroatomes choisis parmi N et O ; éventuellement un cycle non aromatique de 3 à 10 chaînons contenant 1 ou 2 hétéroatomes choisis parmi N et S ; et éventuellement un cycle non aromatique de 3 à 6 chaînons contenant 1 ou 2 hétéroatomes choisis parmi N et S ;
et/ou
« aryle » est un groupe aryle de 6 à 10 chaînons ; éventuellement un groupe phényle ou naphtyle, et éventuellement un groupe phényle, 1-naphtyle ou 2-naphtyle ;
et/ou
« arylène » est un groupe arylène de 6 à 10 chaînons ; et éventuellement un groupe phénylène ou naphtylène ;
et/ou
« hétéroaryle » est un cycle hétéroarylique de 5 à 10 chaînons contenant 1 à 3 hétéroatomes choisis parmi N, O et S ; éventuellement un cycle hétéroarylique de 5 à 10 chaînons contenant 1 à 2 hétéroatomes choisis parmi N, O et S ; éventuellement, le cycle hétéroarylique est choisi parmi un cycle pyridine, un cycle pyrrole, un cycle pyrazole, un cycle pyrimidine, un cycle pyrazine, un cycle pyridazine, un cycle thiophène et un cycle furane ; éventuellement choisi parmi les cycles pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, pyrazin-3-yle, indolyle, isoindolyle, indazolyle, indolizinyle, purinyle, quinolizinyle, quinolinyle, isoquinoléinyle, cinnolinyle, phtalazinyle, naphtyridinyle, quinazolinyle, quinoxalinyle, thiéno[2,3-b]furanyle, furo[3,2-b]pyranyle, pyrido[2,3-d]oxazinyle, pyrazolo[4,3-d]oxazolyle, imidazo[4,5-d]thiazolyle, pyrazino[2,3-d]pyridazinyle, imidazo[2,1-b]thiazolyle, imidazo[1,2-b][1,2,4]triazinyle, benzothiényle, benzoxazolyle, benzimidazolyle, benzothiazolyle, benzoxépinyle, benzoxazinyle, benzofuranyle, benzotriazolyle, pyrrolo[2,3-b]pyridyle, pyrrolo[3,2-c]pyridinyle, pyrrolo[3,2-b]pyridyle, imidazo[4,5-b]pyridyle, imidazo[4,5-c]pyridyle, pyrazolo[4,3-d]pyridyle, pyrazolo[4,3-c]pyridyle, pyrazolo[3,4-c]pyridinyle, pyrazolo[3,4-d]pyridyle, pyrazolo[3,4-b]pyridinyle, imidazo[1,2-a]pyridinyle, pyrazolo[1,5-a]pyridinyle, pyrrolo[1,2-b]pyridazinyle, imidazo[1,2-c]pyrimidinyle, pyrido[3,2-d]pyrimidinyle, pyrido[4,3-d]pyrimidinyle, pyrido[3,4-d]pyrimidinyle, pyrido[2,3-d]pyrimidinyle, pyrido[2,3-b]pyrazinyle, pyrido[3,4-b]pyrazinyle, pyrimido[5,4-d]pyrimidinyle, pyrazolo[2,3-b]pyrazinyle ou pyrimido[4,5-d]pyrimidinyle ; et est éventuellement choisi parmi les cycles pyridin-2-yle, pyridin-3-yle, pyridin-4-yle, pyrimidin-2-yle, pyrimidin-4-yle ou pyrimidin-5-yle ;
et/ou
« hétéroarylène » est un cycle hétéroarylène de 5 à 10 chaînons contenant 1 à 3 hétéroatomes choisis parmi N, O et S ; éventuellement un cycle hétéroaromatique de 5 à 10 chaînons contenant 1 à 2 hétéroatomes choisis parmi N, O et S ; et éventuellement le cycle hétéroarylène est choisi parmi le cycle pyridine, le cycle pyrrole, le cycle pyrazole, le cycle pyrimidine, le cycle pyrazine, le cycle pyridazine, le cycle thiophène, le cycle furane.

3. Composé ou sel de celui-ci selon la revendication 1 ou 2, dans lequel
n₁ est 0 ou 1 ; et/ou n₂ est 1 ; et/ou n₃ est 1 ;
et/ou
R₁ et R₂ sont chacun indépendamment choisis parmi -H, fluor, chlore, brome, hydroxyle, cyano, alkyle en C₁ à C₇ (tel que méthyle, éthyle, n-propyle, isopropyle, n-butyle, isobutyle, tert-butyle, sec-butyle, n-pentyle, 1-méthylbutyle, 2-méthylbutyle, 3-méthylbutyle, isopentyle, 1-éthylpropyle, néopentyle, n-hexyle, 1-méthylpentyle, 2-méthylpentyle, 3-méthylpentyle, isohexyle, 1,1-diméthylbutyle, 2,2-diméthylbutyle, 3,3-diméthylbutyle, 1,2-diméthylbutyle, 1,3-diméthylbutyle, 2,3-diméthylbutyle, 2-éthylbutyle, n-heptyle, 2-méthylhexyle, 3-méthylhexyle, 2,2-diméthylpentyle, 3,3-diméthylpentyle, 2,3-diméthylpentyle, 2,4-diméthylpentyle, 3-éthylpentyle ou 2,2,3-triméthylbutyle), deutéroalkyle en C₁ à C₃ (tel que -CD₃, -C₂D₅ ou -C₃D₇), deutéroalcoxyle en C₁ à C₃ (tel que -OCD₃, -OC₂D₅ ou -OC₃D₇), halogénoalkyle, halogénoalcoxyle, cyclopropanyle, cyclobutanyle, cyclopentanyle ; éventuellement, R₁ est -H ou -CH₃ ; et éventuellement, R₁ est -H, R₂ est -H ;
et/ou
X₁ et X₂ sont chacun indépendamment choisis parmi alkylène en C₁ à C₇ (éventuellement -CH₂-, éthylène, n-propylène, isopropylène, n-butylène ou isobutylène), -O-, -S- ou -NR'- ; éventuellement, X₁ est -CH₂- ou -O- ; éventuellement, X₁ est -O- ; éventuellement, X₂ est -O- ;
éventuellement, R' est choisi parmi -H, alkyle en C₁ à C₇ (éventuellement, méthyle, éthyle, n-propyle, isopropyle, n-butyle, isobutyle, tert-butyle, sec-butyle, n-pentyle, 1-méthylbutyle, 2-méthylbutyle, 3-méthylbutyle, isopentyle, 1-éthylpropyle, néopentyle, n-hexyle, 1-méthylpentyle, 2-méthylpentyle, 3-méthylpentyle, isohexyle, 1,1-diméthylbutyle, 2,2-diméthylbutyle, 3,3-diméthylbutyle, 1,2-diméthylbutyle, 1,3-diméthylbutyle, 2,3-diméthylbutyle, 2-éthylbutyle, n-heptyle, 2-méthylhexyle, 3-méthylhexyle, 2,2-diméthylpentyle, 3,3-diméthylpentyle, 2,3-diméthylpentyle, 2,4-diméthylpentyle, 3-éthylpentyle ou 2,2,3-triméthylbutyle), alkyle deutéré (éventuellement -CD₃, -C₂D₅ ou -C₃D₇) ou cycloalkyle en C₃ à C₆ (éventuellement cyclopropanyle, cyclobutanyle, cyclopentanyle ou cyclohexanyle) ;
et/ou
Ar est un groupe phénylène ou pyridyle, dans lequel les atomes d'hydrogène du phénylène ou pyridyle sont éventuellement substitués par 0, 1, 2 ou 3 substituants, les substituants sont indépendamment choisi parmi F, Cl, Br, I, -CN, -Me, -C₂H₅, cyclopropyle, -CD₃, -OMe, -OCD₃, -CF₃ ou -OCF₃ ; éventuellement, Ar est un groupe phénylène, dans lequel l'atome d'hydrogène dans le groupe phénylène est éventuellement substitué par 2 substituants, et le substituant est F ;
et/ou
Y est -H, -F, -Cl, -Br, méthyle, éthyle, n-propyle, isopropyle, -CD₃, -CF₃, - CH₂CF₃, -OCF₃, -OCHF₂, -OCH₂F, cyclopropyle, cyclobutyle, cyclopentyle, -OCH₃, - OCD₃, -OC₂H₅, -OC₃H₇, ou -OAr' ;
éventuellement,
Y est H, halogène ou -OAr' ; et éventuellement, Y est H, -F ou -OAr' ;
Ar' est choisi parmi phényle, pyridyle, pyrimidyle, pyrrolyle, pyrazolyle, thiényle ou quinoléinyle, et phényle, pyridyle, pyrimidyle, pyrrolyle, pyrazolyle, thiényle ou quinoléine éventuellement,
Ar' est choisi parmi phényle, pyridyle, pyrimidyle, pyrrolyle, pyrazolyle, thiényle ou quinoléinyle, dans lequel les atomes d'hydrogène dans le phényle, pyridyle, pyrimidyle, pyrrolyle, pyrazolyle, thiényle ou quinoléinyle sont chacun indépendamment éventuellement substitués par 1, 2 ou 3 substituants, les substituants sont chacun indépendamment choisis parmi F, Cl, Br, -CN, alkyle en C₁ à C₇ (éventuellement, méthyle, éthyle, n-propyle, isopropyle, n-butyle, isobutyle, tert-butyle, sec-butyle, n-pentyle, 1-méthylbutyle, 2-méthylbutyle, 3-méthylbutyle, isopentyle, 1-éthylpropyle, néopentyle, n-hexyle, 1-méthylpentyle, 2-méthylpentyle, 3-méthylpentyle, isohexyle, 1,1-diméthylbutyle, 2,2-diméthylbutyle, 3,3-diméthylbutyle, 1,2-diméthylbutyle, 1,3-diméthylbutyle, 2,3-diméthylbutyle, 2-éthylbutyle, n-heptyle, 2-méthylhexyle, 3-méthylhexyle, 2,2-diméthylpentyle, 3,3-diméthylpentyle, 2,3-diméthylpentyle, 2,4-diméthylpentyle, 3-éthylpentyle ou 2,2,3-triméthylbutyle), -CD₃, halogénoalkyle en C₁ à C₆, -OCH₃, -OCD₃, -OC₂H₇, -OC₃H₇, halogénoalcoxyle en C₁ à C₆, ou cycloalkyle en C₃ à C₆ (éventuellement cyclopropanyle, cyclobutanyle, cyclopentanyle ou cyclohexanyle) ;
éventuellement,
Ar' est choisi parmi phényle, pyridin-3-yle, pyridin-4-yle ou pyrimidin-5-yle, et est éventuellement substitué par 1 ou 2 substituants, les substituants sont choisis parmi F, Cl, -CH₃, -CF₃ ou -OCF₃ ;
et/ou
Z est O.

4. Composé de formule (I) ou un sel pharmaceutiquement acceptable de celui-ci selon l'une quelconque des revendications 1 à 3, dans lequel le composé de formule (I) est choisi parmi les composés suivants :

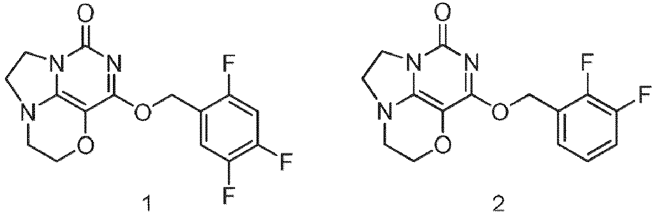

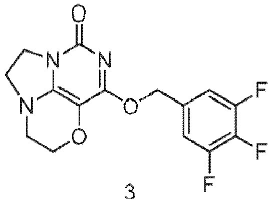

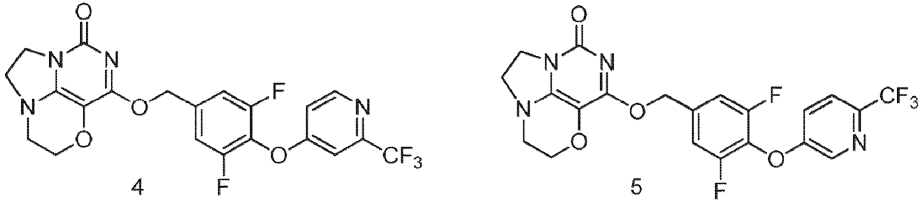

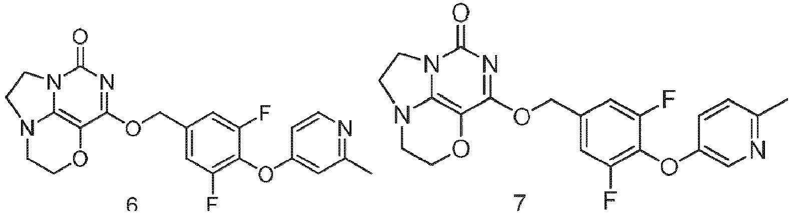

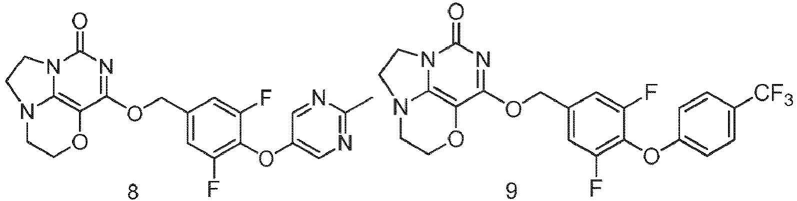

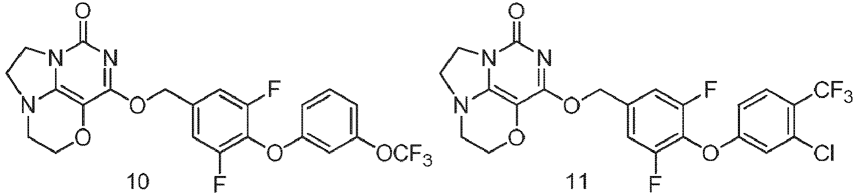

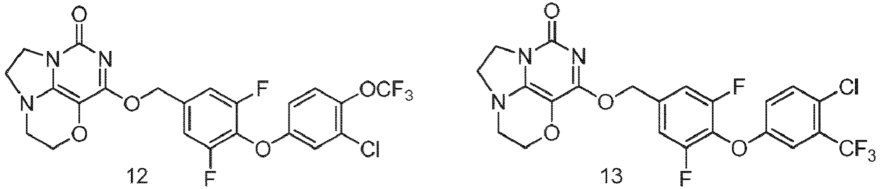

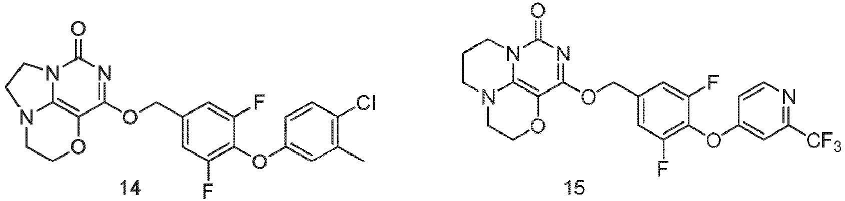

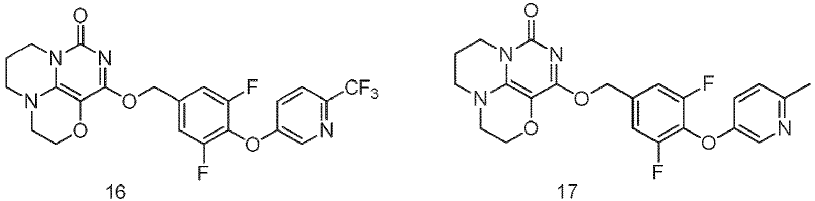

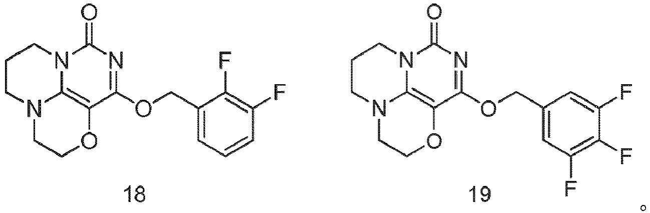

5. Composé de formule (I) ou un sel pharmaceutiquement acceptable de celui-ci selon l'une quelconque des revendications 1 à 4, dans lequel le sel pharmaceutiquement acceptable comprend un sel anionique ou un sel cationique du composé de formule (I) ;
dans lequel le sel pharmaceutiquement acceptable comprend un sel de métal alcalin, un sel de métal alcalino-terreux ou un sel d'ammonium du composé de formule (I) ; éventuellement, le métal alcalin comprend le sodium, le potassium, le lithium ou le césium, et le métal alcalino-terreux comprend le magnésium, le calcium ou le strontium ;
ou le sel pharmaceutiquement acceptable comprend un sel formé par le composé de formule (I) et une base organique ;
éventuellement, la base organique comprend une trialkylamine, la pyridine, la quinoléine, la pipéridine, l'imidazole, la picoline, la diméthylaminopyridine, la diméthylaniline, une N-alkylmorpholine, le 1,5-diazabicyclo[4.3.0]non-5-ène, le 1,8-diazabicyclo[5.4.0]undéc-7-ène, le 1,4-diazabicyclo[2.2.2]octane ; éventuellement, la trialkylamine comprend la triméthylamine, la triéthylamine ou la N-éthyldiisopropylamine ; et éventuellement, la N-alkylmorpholine comprend la N-méthylmorpholine ;
ou le sel pharmaceutiquement acceptable comprend un sel formé par le composé de formule (I) et un acide ;
éventuellement, l'acide comprend un acide inorganique ou un acide organique ; éventuellement, l'acide inorganique comprend l'acide chlorhydrique, l'acide bromhydrique, l'acide iodhydrique, l'acide sulfurique, l'acide nitrique, l'acide phosphorique ou l'acide carbonique ; éventuellement, l'acide organique comprend l'acide formique, l'acide acétique, l'acide propionique, l'acide oxalique, l'acide malonique, l'acide succinique, l'acide fumarique, l'acide maléique, l'acide lactique, l'acide malique, l'acide citrique, l'acide tartrique, l'acide carbonique, l'acide picrique, l'acide méthanesulfonique, l'acide éthanesulfonique, l'acide p-toluènesulfonique, l'acide glutamique ou l'acide pamoïque.

6. Procédé de préparation du composé de formule (I) ou de son tautomère, mésomère, racémate, énantiomère, diastéréoisomère, de sa forme mixte ou de son sel pharmaceutiquement acceptable selon l'une quelconque des revendications 1 à 5, comprenant la voie de synthèse suivante : 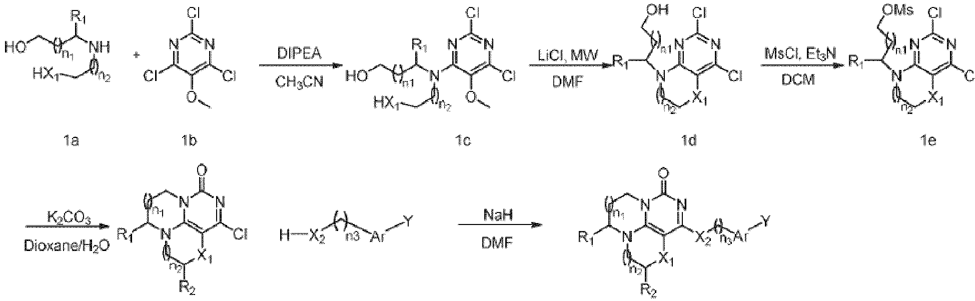 dans chacune des formules du procédé de préparation décrit ci-dessus, n₁, n₂, n₃, R₁, R₂, X₁, X₂, Ar et Y sont définis comme dans l'une quelconque des revendications 1 à 5.

7. Composition pharmaceutique comprenant le composé de formule (I) selon l'une quelconque des revendications 1 à 5, ou son tautomère, mésoforme, racémate, énantiomère, diastéréoisomère ou des mélanges de ceux-ci, ou des sels pharmaceutiquement acceptables de ceux-ci, et éventuellement un véhicule pharmaceutiquement acceptable.

8. Composition pharmaceutique selon la revendication 7, comprenant le composé de formule (I) ou son tautomère, mésomère, racémate, énantiomère, diastéréoisomère ou des mélanges de ceux-ci, ou un ou plusieurs sels pharmaceutiquement acceptables de ceux-ci et éventuellement un véhicule pharmaceutiquement acceptable ;
dans laquelle la formulation orale comprend la formulation solide ou liquide ;
éventuellement, la formulation solide comprend un comprimé, une poudre, un granulée ou une capsule ;
éventuellement, la formulation liquide comprend une suspension aqueuse ou huileuse, ou un sirop ;
éventuellement, la formulation parentérale comprend une solution pour injection ou une suspension aqueuse ou huileuse.

9. Utilisation du composé de formule (I) ou d'un sel pharmaceutiquement acceptable de celui-ci selon l'une quelconque des revendications 1 à 5, ou de la composition pharmaceutique selon la revendication 7 ou 8, dans la préparation d'un inhibiteur de la Lp-PLA2.

10. Utilisation du composé de formule (I) ou d'un sel pharmaceutiquement acceptable de celui-ci selon l'une quelconque des revendications 1 à 5, ou de la composition pharmaceutique selon la revendication 7 ou 8, dans la préparation d'un médicament destiné au traitement de maladies neurodégénératives ;
éventuellement, les maladies neurodégénératives comprennent éventuellement la maladie d'Alzheimer (MA), le glaucome et la dégénérescence maculaire liée à l'âge (DMLA).

11. Utilisation du composé de formule (I) ou d'un sel pharmaceutiquement acceptable de celui-ci selon l'une quelconque des revendications 1 à 5, ou de la composition pharmaceutique selon la revendication 7 ou 8, dans la préparation d'un médicament destiné au traitement de maladies cardiovasculaires, de l'œdème maculaire diabétique (OMD) ou de maladies de la prostate ;
éventuellement, les maladies cardiovasculaires comprennent éventuellement l'athérosclérose.
